# EUROPEAN PATENT APPLICATION

(11) **EP 2 258 713 A1**
(43) Date of publication of application: **08.12.2010**
(21) Application number: 09161734.0
(22) Date of filing: 02.06.2009
(51) Int. Cl.: C07J 41/00, C07H 15/00

(54) **Antiviral Triterpene Derivatives**

(71) Applicant: onepharm Research & Development GmbH, 1210 Wien (AT)
(72) Inventor: Classen-Houben, Dirk, 1150, Vienna (AT); Kueenburg, Bernhard, 1190, Vienna (AT); Kosma, Paul, 1230, Vienna (AT); Jordis, Ulrich, 1180, Vienna (AT); Stanetty, Christian, 1170, Vienna (AT); Szolar, Oliver Dr., 1180 Vienna (AT); Wolkerstorfer, Andrea Dr., 1160 Vienna (AT)
(74) Representative: Redl, Gerda

(57) **Abstract**

The present invention encompasses novel triterpene compounds of general Formula I 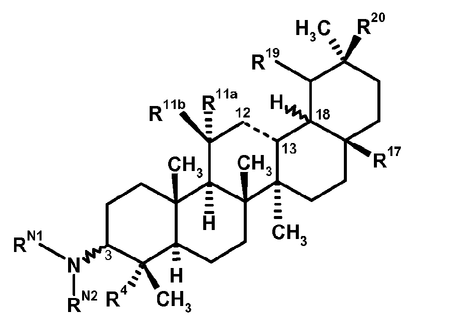 wherein
R^{N1}, R^{N2}, R^{11a}, R^{11b}, R⁴, R¹⁷, R¹⁹ and R²⁰ are defined as in claim 1, which exhibit an antiviral effect and are therefore suitable for the treatment of diseases related thereto and the use thereof for preparing a medicament having the above-mentioned properties.

## Description

### Field of the Invention

This invention relates generally to the field of medicinal chemistry. More specifically, it relates to novel triterpene derivatives, preferably glycyrrhetinic acid derivatives that have pharmacologic activities, formulations containing such and their use to diagnose, cure or prevent certain diseases.

### Background of the Invention

Licorice root is one of the most ancient medical plants being used in the traditional Chinese, Tibetan, Indian and Arabian medicine. The most important, and well-known bioactive component of licorice root is glycyrrhizin (GL), a natural product of the class of triterpene glycosides, also called saponins. Glycyrrhetinic acid (GA) is the aglycone of GL and thus consists only of the triterpene part without the attached sugar molecules (see Figure 1). A variety of pharmacological activities for GL and GA have been reported over the last decades comprising *in vitro* and *in vivo* studies. A good number of publications can be found in the field of steroid metabolism predominantly describing the inhibitory activity for 11β-HSDs with various pharmacological effects. GA has been widely reported as a potent inhibitor of intercellular gap-junctional communication most likely involving connexin43. Furthermore, anti-inflammatory/immunemodulatory effects were reported suggesting several targets involved in the inflammatory process. Several papers report liver protective and anti-cancer properties whereas the impact on apoptosis/oxidative stress has been discussed controversially. Finally, antibiotic and antiviral effects have been reported comprising antibacterial effects on periodontopathogenic bacteria.

The pharmacology and toxicology of GL and GA has been comprehensively reviewed [1-7].

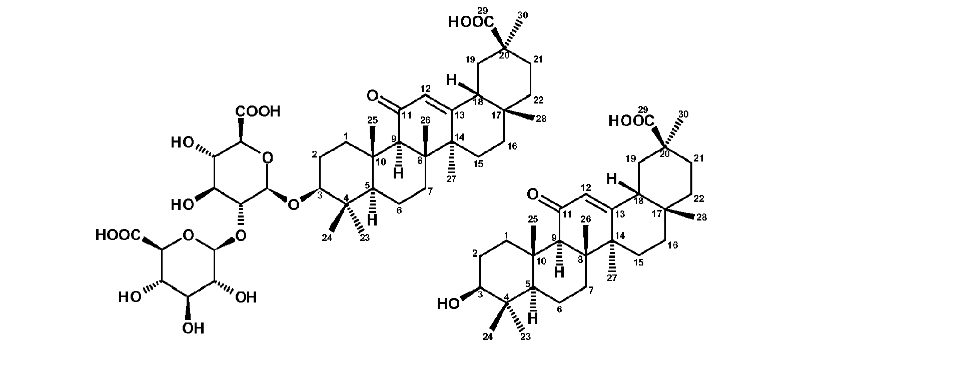

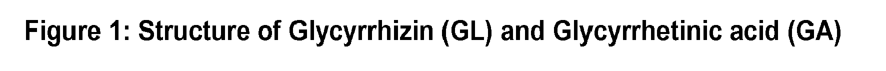

### Impact on Apoptosis and Oxidative Stress

GA is a potent inducer of mitochondrial permeability transition and can trigger the pro-apoptotic pathway. GA is a potent inhibitor of bile acid-induced apoptosis and necrosis in a manner consistent with its antioxidative effect, significantly decreases neutrophil-generated oxygen species and inhibits the generation of inflammatory mediators. Below a certain concentration, GA prevents oxidative stress and mitochondrial permeability transition but at higher concentrations GA induces oxidative stress in certain tissues. GA reveals also an effect on the protein expression of markers of oxidative stress (PAI-1 and p22^{phox}) and scavenges oxygen free radicals in polymorphonuclear leukocytes (PMN). In addition GL stimulates DNA synthesis, proliferation in hepatocytes, and tyrosine phosphorylation of the EGF receptor and p42 MAP kinase.

### Antibiotic and Antiviral Effects

GL, GA and derivatives showed inhibition of replication, growth, proliferation or specific proteins of various viral and bacterial pathogens *in vitro* and *in vivo.* Examples include SARS-coronavirus replication, influenza A virus (H1N1, H2N2, H3N2), herpes simplex virus (HSV), hepatitis C virus, hepatitis A, HIV-1-induced cytopathogenicity, hepatitis B virus (HBV), hyaluronate lyase from *Streptococcus agalactiae,* diverse species of periodontopathogenic and capnophilic bacteria, clarithromycin- and metronidazole-resistant strains of *Heliobacterpylori,* plaque formation in Japan encephalitis virus (JEV), vaccinia virus, Epstein-Barr virus (EBV), and *Leishmania donovani.*

In addition GA and GL result in reduced levels of IL-10 and IL-4, but increased levels of IL-12, IFN-gamma, TNF-alpha, and inducible NO synthase.

### Liver Protective Effects

GA and GL treatment significantly reduces the increase of serum transaminases induced by D-galactosamine (GalN), CC14, or retrorsine. GA inhibits the proliferation and collagen production of hepatic stellate cells (HSCs), down-regulates the mRNA expression of type III and I procollagen, and reduces the deposition of type III and I collagen in fibrotic liver. GA also prevents the depletion of glutathione in the livers of CC14-intoxicated mice and protects gel entrapped hepatocytes from tacrine toxicity.

GA treatment attenuates bile duct and hepatocyte damages in acute vanishing bile duct syndrome (AVBDS) rat model induced by α-naphthylisothiocyanate (ANIT).

### Anti-Inflammatory and Immunmodulatory Effects

GA and GL inhibit secretory type IIA phospholipase A2 purified from the synovial fluids of patients with rheumatoid arthritis. GA inhibits the classical complement pathway at the level of C2, complement C3 is a GL-binding protein and GA induces conformational changes in C3. In the presence of GA, two trypsin-resistent fragments of C3α were immuno-precipitated with anti-C3α which could be selectively purified from the synovial fluids of patients with rheumatoid arthritis. In addition, phosphorylation of C3α by CK-2 was completely inhibited by 30 µM GA. GL (100 µM) induces conformational changes in high mobility group box (HMGB)1 and 2 and completely inhibits the phosphorylation of HMGB1/2 by PKC and CK-I.

GA significantly improved bleeding on probing and gingival inflammation in a clinical study evaluating the local application of a paste containing GA.

The anti-inflammatory activity of GA is similar to hydrocortisone on formalin-induced arthritis in albino rats. Repeated treatment with GA significantly inhibits paw edema of rats with adjuvant arthritis (AA) and croton oil-induced mouse-ear-edema, decreases T-lymphocyte ratio, reduces proliferation of synovial cells and pannus formation, and eliminates the destruction of articular cartilage in inflamed joints of AA rat.

GA suppresses TNFα-induced IL-8 production through blockade in the phosphorylation of MAPKs, following IκBα degradation and NFκB activation. GL enhances interleukin-2 (IL-2) secretion and IL-2 receptor (IL-2R) expression. In addition GL promotes tyrosine phosphorylation of p56 induced by anti-CD3. GL augments lipopolysaccharide (LPS)-induced IL-12 p40 mRNA expression, transcription of IL-12 mRNAs and IL-12-protein production. GL increases production of IL-10 *in vitro* and in mice with Con A-induced hepatitis. GL inhibits prostaglandin E2 production and release of [3H]arachidonic acid. GA lowers inflammatory capillary permeability, inhibits neutrophil emigration and prostaglandin E2 synthesis, and scavenges free radicals in a rat model of histamine, carrageenan, or ararachidonic acid-induced peritonitis. GA dose-dependently increases NO production and iNOS mRNA through activation of protein/DNA binding of NF-κB to its cognate site, enhances the production of nitric oxide from IFN-γ activated cells and tumor cell killing by macrophages activated with IFN-γ. This tumor cell killing is mainly by nitric oxide.

Anti-inflammatory activities of natural triterpenoids including GA have been reviewed recently.

### Short Chain Dehydrogenase Reductases (SDR) and Corticoid Metabolism

GA is a potent non-competitive inhibitor of different hydroxysteroid dehydrogenases (HSD). GA inhibits 11β-HSD 1 and 11β-HSD 2 involved in the metabolism of corticosteroids, 3α-HSD involved in inflammatory processes, 3α/β,20β-HSD involved in the metabolism of androgens and progestins, 5β-HSD involved in the metabolism of cortisol, aldosterone and testosterone, and 3β-HSD involved in the metabolism of aldosterone and other steroids.

GL and GA can bind to mineralocorticoid and glucocorticoid receptors with low but sufficient affinity in order to explain the mineralocorticoid-like side effects. GA potentiates the action of aldosterone and facilitates the active transport of sodium in frog skin epithelium. GA stimulates an increase in steroid production in adrenal cells lacking intact cell junctions.

Especially the modification of corticosteroid levels by inhibition of 11β-HSD 1 and 2 by GA has been connected to numerous biological states and diseases. Examples include the reversible, gradual, constant and significant increase in systolic blood pressure, reduction in diuresis and increase in renal sodium retention, the reduction of thigh circumference and thickness of the subcutaneous fat layer in human volunteers after topical application, the reduction of metabolic detoxification of the cigarette smoke carcinogen nitrosamine 4-methylnitrosamino-1-(3-pyridyl)-1-butanone (NNK), the involution of the thymus and thymocyte apoptosis, the potentiation of corticosteroid effects in cultured primary human bronchial epithelial cells (PBECs), ear swelling in dinitrofluorobenzene challenged mice, human volunteer skin vasoconstrictor assay and lung tissue, the retardation of the development of autoimmune disease, as well as the increased glucose use in subregions of the hypothalamus, hippocampus, neocortex and subthalamus.

11β-HSD mRNA is expressed in neurones of the hypothalamic paraventricular nucleus (PVN) where corticotrophin-releasing factor-41 (CRF-41) is synthesized and GA decreases the release of CRF-41 into hypophysial portal blood in rats, suggesting that 11β-HSD regulates the effective corticosterone feedback signal to CRF-41 neurons.

### Anticancer Effects

GA inhibits oxidative stress DMBA/TPA-induced skin tumor formation, inhibits ear edema and ornithine decarboxylase activity induced by croton oil in mice, protects against rapid DNA damage and decreases unscheduled DNA synthesis induced by benzo[a]pyrene, increases the antiproliferative effect of glucocorticoids In MCF-7 and ZR-75-1 breast cancer cells, reduces the tumor weight in rats transplanted with 'Oberling-Guerin' myeloma, inhibits proliferation of HepG2 human hepatoma cell line, inhibits the mutagenicity of benzo[a]pyrene, 2-aminofluorene and aflatoxin B1, and protects against tumor initiation as well as tumor promotion by 7,12-dimethylbenz[a]anthracene (DMBA) and 12-O-tetradecanoylphorbol-13-acetate.

GA also increases the accumulation of calcein, a fluorescent substrate of multidrug resistance protein 1 (MRP1) and of daunorubicin, a fluorescent substrate of P-glycoprotein, resulting in sensitivity to anticancer drugs, showing that GA reverses multidrug resistance.

### Gap Junction Blockade and Endothelial Relaxation

GA inhibits intercellular gap-junctional communication in human fibroblasts and cultured rat neonatal cardiomyocytes, as well as type 1 or type 2A protein phosphatase-mediated Connexin43 dephosphorylation in WB-F344 rat liver epithelial cells. GA inhibits fluorescence replacement after photobleaching (FRAP) in primary chick osteocyte cultures, also indicating gap junction blockade.

GA increases the apparent cell input resistance and completely blocks membrane chloride conductance blocked while Na⁺ and K⁺ conductance are virtually unchanged.

GA in a concentration-dependent fashion attenuates EDHF-type relaxations to acetylcholine (ACh), observed in the presence of NG-nitro-L-arginine methyl ester (L-NAME) and indomethacin, modulates contractions produced by nor-adrenalin or high-K solutions and significantly reduces ACh-induced hyperpolarizations in both, endothelial and smooth muscle cells of guinea pig coronary and rat mesenteric arteries. Inhibition of the EDHF-hyperpolarization and relaxation in the smooth muscle may stem from the inhibition of endothelial cell hyperpolarization. GA quickly blocked electrical communication between smooth muscle and endothelial cells in guinea-pig mesenteric arterioles.

GA inhibits pressure-induced myogenic tone of rat middle cerebral arteries and vasopressin-induced vasoconstriction, increases input resistance in rat isolated mesenteric small arteries, desynchronised isolated smooth muscle cells, and had nonjunctional effects on membrane currents. GA significantly increases the frequency of phrenic bursts decreases the peak amplitude of integrated phrenic nerve discharge in an arterially perfused rat preparation.

GA inhibits the spike component of the action potential (AP), reduces contraction evoked by electrical stimulation, inhibits slow depolarization with superimposed APs and phasic contractions of the ureter induced by neurokinin A, and inhibits the KCI-evoked APs and phasic contractions without affecting the sustained responses in the guinea pig ureter.

GA inhibits frequencies of paced contractions, likely owing to inhibition of I-type Ca²⁺ channels, reduces the amplitudes of spontaneous and nerve-induced contractions, decreases phasic contractions and depolarizes resting membrane potential in murine small intestinal muscles. GA also inhibits the spread of Lucifer yellow, increases input resistance, decreases cell capacitance in interstitial cells of Cajal networks and decreased L-type Ca²⁺ current without affecting the voltage dependence of this current.

GA decreased the postsynaptic light response in murine retinal ganglion cells to 30 % of control.

### Other Effects

GA reduces the bon resorption in rheumatoid arthritis and periodontits.

GA reduces coughing in guinea-pigs by 50 % compared to saline.

GA increases cytoplasmic free Ca²⁺ and inhibits Ca²⁺ increases induced by antigen, ATP, phenyephrine and thrombin. GA inhibits dexamethasone-induced increases in the histamine synthesis and histamine release. GA inhibits histidine decarboxylase and maturation of mast cells, lowers expression of PKC delta mRNA suggesting that the inhibition of histamine synthesis by GA is regulated by nPKC delta. GA significantly inhibits the degranulation of RBL-2H3 cells induced by IgE with the antigen (DNP-HSA) and rat peritoneal mast cells induced by compound 48/80. GA inhibits the passive cutaneous anaphylactic reaction as well as the scratching behaviour in mice induced by compound 48/80 and the production of IgE in ovalbumin-induced asthma mice.

GA sodium salt strongly counteracts arrhythmia induced by chloroform, lengthens the appearance time of arrhythmia induced by CaCl₂, slightly retards the heart rate of rats and rabbits, and partly antagonizes the acceleration effect of isoproterenol on rabbit hearts.

GA competitively inhibits the Na⁺/K⁺-ATPase of canine kidney basolateral membranes.

GA significantly increases insulin-stimulated glucose uptake in 3T3-L1 adipocytes, glucose-stimulated insulin secretion in islets isolated from mice and induces mRNA levels of insulin receptor substrate-2, pancreas duodenum homeobox-1 and glucokinase in islets.

### Summary of the Invention

New triterpene derivatives, preferably glycyrrhetinic acid derivatives have been prepared and show desirable biological and pharmacologic activities relevant for the diagnosis, prevention and therapy of certain diseases.

Accordingly, in one its aspects, the present invention includes a compound selected from a compound of Formula I: 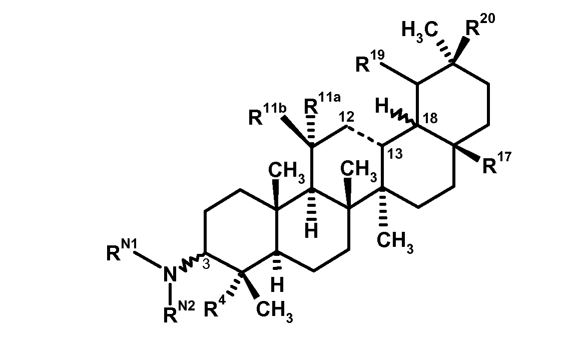 wherein
**R^{N1}** is selected from hydrogen, **-R^{a},** -O-**R^{a},** -C(=O)-**R**^{a}; and
**R^{N2}** is selected from -**R^{b}**, -(CH₂)ₙ-NH-**R^{b}**, -C(=O)(CH2)n-N(-O-**R^{a}**)-**R^{b}**, -(CH₂)ₙ-N(-O-**R^{a}**)-**R^{b}**, -(CH₂)ₙ-O-**R^{b},** -(CH₂)ₙ-S-**R^{b}**; and
**R^{11a}** and **R^{11b}** together are selected from =O, =N-**R^{a}**, =N-O-**R^{a}**; or
**R^{11a}** and **R^{11b}** are independently from one another selected from hydrogen, -O-R^{a}, -O-C(=O)-R^{a}, -NH-R^{a}, methyl, ethyl, ethynyl, fluorine, chlorine, and bromine with the proviso, that **R^{11a}** and **R^{11b}** are not both hydrogen; and
a single or double bond is present at **12-13;**
**R⁴, R¹⁷, R¹⁹** and **R²⁰** are independently from one another selected from hydrogen, methyl, -CH₂-O-**R^{a}**, -CHO, -CO₂-**R^{a}**; and
**R^{a}** is selected from hydrogen, benzhydryl, or from optionally substituted carboxylic acid, C₁₋₆alkyl, C₂₋₆alkenyl, C₂₋₈alkynyl, -CF₃, -(CH₂)ₙ-C₆₋₁₄aryl, -CH=CH-C₆₋₁₄aryl, -C≡C-C₆₋₁₄aryl, -(CH₂)ₙ-C₅₋₁₄heteroaryl, -CH=CH-C₅₋₁₄heteroaryl, -C≡C-C₅₋₁₄heteroaryl,-(CH₂)ₙ-C₃₋₈Cycloalkyl, -CH=CH-C₃₋₈cycloalkyl and -C=C-C₃₋₈cycloalkyl; and
**R^{b}** is selected from an optionally substituted sugar; and
each **n** independently of one another denotes 0, 1 2, 3, 4, or 5; and optionally in the form of the pharmaceutically effective salts, solvates, prodrugs, tautomers, racemates, enantiomers, diastereomers or mixtures thereof.

One aspect of the invention relates to compounds of general Formula I, wherein the configuration at atom **3** and **18** can be independently from one another either R or S.

One aspect of the invention relates to compounds of general Formula I, wherein **R⁴, R¹⁷** and **R¹⁹** denote methyl.

Another aspect of the invention relates to compounds of general Formula I, wherein **R²⁰** denotes **-CO₂-R^{a}.**

Another aspect of the invention relates to compounds of general Formula I, wherein **R^{11a}** and **R^{11b}** together denote =O; and a double bond is present at 12-13.

Another aspect of the invention relates to compounds of general Formula I, wherein **R^{N2}** is selected from **-R^{b},** -(CH₂)ₙ-S-**R^{b}**, and -C(=O)(CH₂)ₙ-N(-O-**R^{a}**)-**R^{b}**

Another aspect of the invention relates to compounds of general Formula I, wherein **R^{b}** is selected from optionally substituted β-D-glucopyranosiduronic acid, β-D-glucopyranose, α-L-rhamnopyranose, β-D-galactopyranose, β-D-xylopyranose, α-L-arabinofuranose, β-D-quinovopyranose, β-D-apiofuranose, gentibiose, chinovopyranose, fucopyranosyl, β-D-galactopyranosiduronic acid, β-D-ribopyranose, β-D-xylofuranose, β-D-allopyranose, β-D-arabinopyranose and β-D-apiopyranose.

Another aspect of the invention relates to compounds of general Formula I, wherein **R^{N1}** is selected from hydrogen or from optionally substituted methoxy, acetyl and succinyl.

Another aspect of the invention relates to a compound of general Formula I which is selected from the group consisting of
(3S,18R,20S)-11-oxo-3-[(2,3,4,6-tetra-*O*-acetyl-beta-D-glucopyranosyl) methoxamino]-olean-12-en-29-oic acid, diphenylmethyl ester,
(3S,18R,20S)-11-oxo-3-[(2,3,4,6-tetra-*O*-acetyl-beta-D-glucopyranosyl) methoxamino]-olean-12-en-29-oic acid,
(3S,18R,20S)-11-oxo-3-[(beta-D-glucopyranosyl)methoxamino]-olean-12-en-29-oic acid,
(3S,18R,20S)-3-[2-S-(methyl-2,3,4-tri-*O*-acetyl-1-thio-beta-D-glucopyranosyluronate)-ethyl-amino]-11-oxo-olean-12-en-29-oic acid, diphenylmethyl ester,
(3S,18R,20S)-3-[2-S-(methyl-2,3,4-tri-*O*-acetyl-1-thio-beta-D-glucopyranosyluronate)-ethyl-amino]-11-oxo-olean-12-en-29-oic acid,
(3S,18R,20S)-3-[2-S-(methyl-1-thio-beta-D-glucopyranosyluronate)-ethyl-amino]-11-oxo-olean-12-en-29-oic acid,
(3S,18R,20S)-3-[2-(beta-D-glucopyranuronosylthio)-ethyl-amino]-11-oxo-olean-12-en-29-oic acid,
(3S,18R,20S)-3-{*N*-acetyl-N-[2-S-(methyl-2,3,4-tri-*O*-acetyl-1-thio-beta-D-glucopyranosyluronate)-ethyl]-amino}-11-oxo-olean-12-en-29-oic acid, diphenylmethyl ester,
(3S,18R,20S)-3-{*N*-acetyl-N-[2-S-(methyl-2,3,4-tri-*O*-acetyl-1-thio-beta-D-glucopyranosyluronate)-ethyl]-amino}-11-oxo-olean-12-en-29-oic acid,
(3S,18R,20S)-3-{*N*-acetyl-*N*-[2-S-(methyl-1-thio-beta-D-glucopyranosyluronate)-ethyl]-amino}-11-oxo-olean-12-en-29-oic acid,
(3S,18R,20S)-3-{*N*-acetyl-*N*-[2-(beta-D-glucopyranuronosylthio)-ethyl]-amino}-11-oxo-olean-12-en-29-oic acid,
(3S,18R,20S)-3-{*N*-(4-methoxy-1,4-dioxobutyl)-N-[2-S-(methyl-2,3,4-tri-*O*-acetyl-1-thio-beta-D-glucopyranosyluronate)-ethyl]-amino}-11-oxo-olean-12-en-29-oic acid, diphenylmethyl ester,
(3S,18R,20S)-3-{*N*-(4-methoxy-1,4-dioxobutyl)-N-[2-S-(methyl-2,3,4-tri-*O*-acetyl-1-thio-beta-D-glucopyranosyluronate)-ethyl]-amino}-11-oxo-olean-12-en-29-oic acid,
(3S,18R,20S)-3-{*N*-(4-methoxy-1,4-dioxobutyl)-N-[2-S-(methyl-1-thio-beta-D-glucopyranosyluronate)-ethyl]-amino}-11-oxo-olean-12-en-29-oic acid and
(3S,18R,20S)-3-{*N*-(4-methoxy-1,4-dioxobutyl)-N-[2-(beta-D-glucopyranurosylthio)-ethyl]-amino}-11-oxo-olean-12-en-29-oic acid

One aspect of the invention related to compounds of general Formula I, or the pharmacologically effective salts thereof, as medicaments.

One aspect of the invention relates to pharmaceutical preparation, containing as active substance one or more compounds of general Formula I, or the pharmacologically effective salts thereof, optionally in combination with conventional excipients and/or carriers.

One aspect of the invention is the use of compounds of general Formula I for preparing a medicament for the treatment and/or prevention of chronic inflammatory diseases, autoimmune diseases, skin diseases, bone diseases, metabolic diseases, infectious diseases and cancer.

One aspect of the invention is the use of compounds of general Formula I for preparing a medicament for the treatment and/or prevention of infectious diseases.

In a further aspect, the present invention includes a use of a compound or a combination of compounds of the invention as a medicament or as a diagnostic.

In a further aspect, the present invention includes use of a compound or a combination of compounds of the invention to treat a condition or disease that benefits from the inhibition of 11β-HSD isozymes. In particular embodiments the condition or disease that benefit from 11β-HSD inhibition are chronic inflammatory diseases.

Accordingly, also included within the scope of the present invention is a method of treating chronic inflammatory diseases, comprising administering an effective amount of a compound of the invention to a subject in need thereof. Further the invention includes a use of a compound of the invention to treat chronic inflammatory diseases, as well as a use of a compound of the invention to prepare a medicament to treat chronic inflammatory diseases.

In a further aspect, the present invention includes a method of treating autoimmune diseases, comprising administering an effective amount of a compound of the invention to a subject in need thereof. Further the invention includes a use of a compound of the invention to treat autoimmune diseases, as well as a use of a compound of the invention to prepare a medicament to treat autoimmune diseases.

Also included within the scope of the present invention is a method of treating skin diseases, comprising administering an effective amount of a compound of the invention to a subject in need thereof. Further the invention includes a use of a compound of the invention to treat skin diseases, as well as a use of a compound of the invention to prepare a medicament to treat skin diseases.

The present invention also includes a method of treating metabolic diseases, comprising administering an effective amount of a compound of the invention to a subject in need thereof. The invention also includes a use of an 11β-HSD inhibiting compound of the invention to treat metabolic diseases, as well as a use of an 11β-HSD inhibiting compound of the invention to prepare a medicament to treat and metabolic diseases.

An additional aspect of the present invention is a method of treating infectious diseases comprising administering an effective amount of a compound of the invention to a subject in need thereof. Also included in the present invention is a use of a compound of the invention to treat infectious diseases as well as a use of a compound of the invention to prepare a medicament to treat infectious diseases.

A further aspect of the present invention is a method of treating cancer comprising administering an effective amount of a compound of the invention to a subject in need thereof. Also included in the present invention is a use of a compound of the invention to treat cancer as well as a use of a compound of the invention to prepare a medicament to treat cancer.

Other features and advantages of the present invention will become apparent from the detailed description. It should be understood, however, that the detailed description and the specific examples while indicating preferred embodiments of the invention are given by way of illustration only, since various changes and modifications within the spirit and scope of the invention will become apparent to those skilled in the art from this detailed description.

### Detailed Description of the Invention

The compounds of the invention include compounds of Formula I as hereinbefore defined, including all polymorphs and crystal habits thereof, salts, prodrugs and isomers thereof (including optical, geometric and tautomeric isomers) as hereinafter defined and isotopically-labeled compounds of Formula I.

Unless specified otherwise, the term *alkyl,* when used alone or in combination with other groups or atoms, refers to a saturated straight or branched chain consisting solely of 1 to 6 hydrogen-substituted carbon atoms, and includes methyl, ethyl, propyl, isopropyl, n-butyl, 1-methylpropyl, isobutyl, t-butyl, 2,2-dimethylbutyl, n-pentyl, 2-methylpentyl, 3-methylpentyl, 4-methylpentyl, n-hexyl and the like.

Unless specified otherwise, the term *alkenyl* refers to a partially unsaturated straight or branched chain consisting solely of 2 to 6 hydrogen-substituted carbon atoms that contains at least one double bond, and includes vinyl, allyl, 2-methylprop-1-enyl, but-1-enyl, but-2-enyl, but-3-enyl, buta-1,3-dienyl, penta-1,3-dienyl, penta-2,4-dienyl, 2-methylbut-1-enyl, 2-methylpent-1-enyl, 4-methylpent-1-enyl, 4-methylpent-2-enyl, 2-methylpent-2-enyl, 4-methylpenta-1,3-dienyl, hexen-1-yl and the like.

Unless specified otherwise, the term *alkynyl* refers to a partially unsaturated straight or branched chain consisting solely of 2 to 8 hydrogen-substituted carbon atoms that contains at least one triple bond, and includes ethynyl, 1-propynyl, 2-propynyl, 2-methylprop-1-ynyl, 1-butynyl, 2-butynyl, 3-butynyl, 1,3-butadiynyl, 3-methylbut-1-ynyl, 4-methylbut-ynyl, 4-methylbut-2-ynyl, 2-methylbut-1-ynyl, 1-pentynyl, 2-pentynyl, 3-pentynyl, 4-pentynyl, 1 ,3-pentadiynyl, 1,4-pentadiynyl, 3-methylpent-1-ynyl, 4-methylpent-2-ynyl, 4-methylpent-2-ynyl, 1-hexynyl, and the like.

Unless specified otherwise, the term *hydroxyalkyl* refers to an aliphatic alkyl-, alkenyl-, or alkynyl-groups substituted with one or more hydroxyl-groups, and includes 2-hydroxyethyl, 2-hydroxypropyl, 3-hydroxypropyl, 2-hydroxybutyl, 3-hydroxybutyl, 4-hydroxybutyl, 2-hydroxy-1-methyl-ethyl, 2-hydroxy-1-ethyl-ethyl, 2,3-bihydroxypropyl, 2,3,4-trihydroxybutyl, 2-hydroxy-1-hydroxymethyl-ethyl, 3-hydroxy-2-hydroxymethyl-propyl, 3-hydroxy-1-(2-hydroxyethyl)-propyl, and the like.

Unless specified otherwise, the term *carboxylic acid* refers to an aliphatic mono-or dicarboxylic acid group containing from 2 to 18 carbon atoms that may optionally be substituted with one or more, identical or different substituents, independently selected from C₁₋₄alkyl, fluoro-substituted C₁₋₄alkyl, halo, OC₁₋₄alkyl, fluoro-substituted OC₁₋₄alkyl, aryl, heteroaryl, NH₂, NH(alkyl), N(alkyl)₂, CO₂H, CO₂(alkyl), NO₂ and CN. Examples of carboxylic acids include formic acid, acetic acid, propionic acid, butyric acid, valeric acid, caproic acid, enanthic acid, caprylic acid, acrylic acid, pyruvic acid, acetoacetic acid, oxalic acid, malonic acid, malic acid, fumaric acid, succinic acid, glutaric acid, adipic acid, lactic acid, and the like.

Unless specified otherwise, the term *carboxylic acid ester* refers to alkylesters, arylesters, alkenylesters, alkynylester, cycloalkylesters, hydroxyalkylesters, and the like of above defined carboxylic acids. Examples of carboxylic acid esters include methylesters, ethylesters, isopropylesters, t-butylesters, benzylesters, benzhydrylesters, allylesters, and the like.

Unless specified otherwise, the term *cycloalkyl,* when used alone or in combination with other groups or atoms, refers to a saturated or unsaturated ring consisting solely of 3 to 8 carbon atoms, that may optionally be substituted with one or more, identical or different substituents, suitably one to three substituents, independently selected from C₁₋₄alkyl, fluoro-substituted C₁₋₄alkyl, halo, OC₁₋₄alkyl, fluoro-substituted OC₁₋₄alkyl, NH₂, NH(alkyl), N(alkyl)₂, CO₂H, CO₂(alkyl), NO₂ and CN. Examples of cycloalkyl groups include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, cyclooctenyl and the like.

Unless specified otherwise, the term *aryl* refers to an aromatic mono- or bicyclic group containing from 6 to 14 carbon atoms that may be optionally fused with a fully or partially saturated or unsaturated carbocyclic ring and may optionally be substituted with one or more, identical or different substituents, suitably one to three substituents, independently selected from C₁₋₄alkyl, fluoro-substituted C₁₋₄alkyl, halo, OC₁₋₄alkyl, fluoro-substituted OC₁₋₄alkyl, NH₂, NH(alkyl), N(alkyl)₂, CO₂H, CO₂(alkyl), NO₂and CN. Examples of aryl groups include phenyl, naphthyl, indanyl, and the like.

Unless specified otherwise, the term *heteroaryl* refers to an aromatic mono- or bicyclic group containing from 5 to 14 carbon atoms, of which one to five is replaced with a heteroatom selected from N, S and O, that may optionally be reduced to a nonaromatic heterocycle and may optionally be substituted with one or more, identical or different substituents, suitably one to three substituents, independently selected from C₁₋₄alkyl, fluoro-substituted C₁₋₄alkyl, halo, OC₁₋₄alkyl, fluoro-substituted OC₁₋₄alkyl, NH₂, NH(alkyl), N(alkyl)₂, CO₂H, CO₂(alkyl), NO₂ and CN. Examples of heteroaryl groups include pyrrolyl, dihydropyrrolyl, pyrrolidinyl, indolyl, isoindolyl, indolizinyl, imidazolyl, pyrazolyl, benzimidazolyl, imidazo(1,2-a)pyridinyl, indazolyl, purinyl, pyrrolo(2,3-c)pyridinyl, pyrrolo(3,2-c)pyridinyl, pyrrolo(2,3-b)pyridinyl, pyrazolo(1,5-a)pyridinyl, 1,2,3-triazolyl, 1,2,4-triazolyl, tetrazolyl, oxazolyl, isoxazolyl, 1,3,4-oxadiazolyl, 1,2,5-oxadiazolyl, 1,2,4-oxadiazolyl, 1,2,3-oxadiazolyl, thiazolyl, isothiazolyl, 1,3,4-thiadiazolyl, 1,2,5-thiadiazolyl, 1,2,4-thiadiazolyl, 1,2,3-thiadiazolyl, furanyl, dihydrofuranyl, tetrahydrofuranyl, benzofuranyl, isobenzofuranyl, thiophenyl, dihydrothiophenyl, tetrahydrothiophenyl, benzothiophenyl, benzoisothiophenyl, pyridyl, piperidinyl, quinolinyl, isoquinolinyl, quinolizinyl, pyrazinyl, pyridazinyl, pyrimidinyl, pyranyl, tetrahydropyranyl, 1,2,3-triazinyl, 1,2,4-triazinyl, 1,3,5-triazinyl, chromenyl, morpholinyl, diazepinyl, benzodiazepinyl, and the like.

Unless specified otherwise, the term *sugar,* when used alone or in combination with other groups or atoms, refers to naturally occurring or chemically modified carbohydrates that might be connected via glycosidic bonds to a free hydroxyl group of additional sugars and may optionally be substituted with one or more, identical or different substituents, suitably one to three substituents. Examples of sugars include but are not limited to β-D-glucospyranose, α-L-rhamnopyranose, β-D-galactopyranose, β-D-xylopyranose, α-L-arabinofuranose, β-D-quinovopyranose, β-D-apiofuranose, gentibiose, chinovopyranose, fucopyranosyl, β-D-glucopyranosiduronic acid, β-D-galactopyranosiduronic acid, β-D-ribopyranose, β-D-xylofuranose, β-D-allopyranose, β-D-arabinopyranose, β-D-apiopyranose and the like.

Unless specified otherwise, the term *fluoro-substituted* as used herein means that, in the group being described, one or more, including all, of the hydrogen atoms has been replaced by F. For example, a fluoro-substituted alkyl includes trifluoromethyl, trifluoroethyl, pentafluoroethyl and the like.

Unless specified otherwise, as used herein, the terms *halogen* and *halo* include F, Cl, Br, and I. Under standard nomenclature rules used throughout this disclosure, the point of attachment of the designated side chain is described first followed by the adjacent functionality toward the terminal portion. A substituent's point of attachment may also be indicated by a dashed line to indicate the point(s) of attachment, followed by the adjacent functionality and ending with the terminal functionality.

It is intended that the definition of any substituent or variable at a particular location in a molecule be independent of its definitions elsewhere in that molecule. It is understood that substituents and substitution patterns on the compounds of this invention can be selected by one of ordinary skill in the art to provide compounds that are chemically stable and that can be readily synthesized by techniques known in the art as well as those methods set forth herein.

The term *pharmaceutically acceptable* means compatible with the treatment of animals, in particular, humans. The term *pharmaceutically acceptable salt* includes both pharmaceutically acceptable acid addition salts and pharmaceutically acceptable basic addition salts.

The term *pharmaceutically acceptable acid addition salt* as used herein means any non-toxic organic or inorganic salt of any base compound of the disclosure, or any of its intermediates. Basic compounds of the disclosure that may form an acid addition salt include, for example, compounds that contain a basic nitrogen atom. Illustrative inorganic acids which form suitable salts include hydrochloric, hydrobromic, sulfuric and phosphoric acids, as well as metal salts such as sodium monohydrogen orthophosphate and potassium hydrogen sulfate. Illustrative organic acids that form suitable salts include mono-, di-, and tricarboxylic acids such as glycolic, lactic, pyruvic, malonic, succinic, glutaric, fumaric, malic, tartaric, citric, ascorbic, maleic, benzoic, phenylacetic, cinnamic and salicylic acids, as well as sulfonic acids such as p-toluene sulfonic and methanesulfonic acids. Either the mono-, di- or the tri-acid salts can be formed, and such salts may exist in either a hydrated, solvated or substantially anhydrous form. In general, the acid addition salts of the compounds of the disclosure are more soluble in water and various hydrophilic organic solvents, and generally demonstrate higher melting points in comparison to their free base forms. The selection of the appropriate salt will be known to one skilled in the art. Other non-pharmaceutically acceptable acid addition salts, e.g. oxalates, may be used, for example, in the isolation of the compounds of the disclosure, for laboratory use, or for subsequent conversion to a pharmaceutically acceptable acid addition salt.

The term *pharmaceutically acceptable basic salt* as used herein means any non-toxic organic or inorganic basic addition salt of any acid compound of the invention, or any of its intermediates, which are suitable for or compatible with the treatment of animals, in particular humans. Acidic compounds of the invention that may form a basic addition salt include, for example compounds that contain carboxylic acid, sulfonic acid, sulfinic acid, sulfonamide, N-unsubstituted tetrazole, phosphoric acid ester, or sulfuric acid ester. Illustrative inorganic bases which form suitable salts include lithium, sodium, potassium, calcium, magnesium, or barium hydroxide. Illustrative organic bases which form suitable salts include aliphatic, alicyclic or aromatic organic amines such as methylamine, trimethylamine and picoline or ammonia. The selection of the appropriate salt will be known to a person skilled in the art. Other non-pharmaceutically acceptable basic addition salts, may be used, for example, in the isolation of the compounds of the invention, for laboratory use, or for subsequent conversion to a pharmaceutically acceptable acid addition salt. The formation of a desired compound salt is achieved using standard techniques. For example, the neutral compound is treated with a base in a suitable solvent and the formed salt is isolated by filtration, extraction or any other suitable method.

The term *subject* or *patient* or synonym thereto, as used herein includes all members of the animal kingdom, especially mammals, including human. The subject or patient is suitably a human.

As used herein, and as well understood in the art, *treatment* is an approach for obtaining beneficial or desired results, including clinical results. Beneficial or desired clinical results can include, but are not limited to, alleviation or amelioration of one or more symptoms or conditions, diminishment of extent of disease, stabilized (i.e. not worsening) state of disease, preventing spread of disease, delay or slowing of disease progression, amelioration or palliation of the disease state, and remission (whether partial or total), whether detectable or undetectable. *Treatment* can also mean prolonging survival as compared to expected survival if not receiving treatment.

*Palliating* a disease or disorder means that the extent and/or undesirable clinical manifestations of a disorder or a disease state are lessened and/or time course of the progression is slowed or lengthened, as compared to not treating the disorder. The term *prevention* or *prophylaxis,* or synonym thereto, as used herein refers to a reduction in the risk or probability of a patient becoming afflicted with the disease or manifesting a symptom associated with the disease.

The term *therapeutically effective amount, effective amount* or *sufficient amount* of a compound of the present invention is a quantity sufficient to, when administered to the subject, including a mammal, for example a human, effect beneficial or desired results, including clinical results, and, as such, an *effective amount* or synonym thereof depends upon the context in which it is being applied. For example, in the context of 11β-HSD inhibition, it is an amount of the compound sufficient to achieve an inhibition of 11β-HSD activity compared to the response obtained without administration of the compound. In the context of disease, therapeutically effective amounts of the compounds of the present invention are used to treat, modulate, attenuate, reverse, or affect a disease or conditions that benefits from an inhibition of 11β-HSD, for example, chronic inflammatory diseases. An *effective amount* is intended to mean that amount of a compound that is sufficient to treat, prevent or inhibit such diseases or conditions. The amount of a given compound of the present invention that will correspond to such an amount will vary depending upon various factors, such as the given drug or compound, the pharmaceutical formulation, the route of administration, the type of disease or disorder, the identity of the subject or host being treated, and the like, but can nevertheless be routinely determined by one skilled in the art. Also, as used herein, a *therapeutically effective amount* of a compound of the present invention is an amount which prevents, inhibits, suppresses or reduces a disease or conditions that benefits from an inhibition of 11β-HSD, for example, chronic inflammatory diseases as determined by clinical symptoms in a subject as compared to a control. As defined herein, a therapeutically effective amount of a compound of the present invention may be readily determined by one of ordinary skill by routine methods known in the art.

In an embodiment, a therapeutically effective amount of a compound of the present invention ranges from about 0.01 to about 100 mg/kg body weight, suitably about 0.02 to about 50 mg/kg body weight, and more suitably, from about 0.05 to about 20 mg/kg body weight. The skilled artisan will appreciate that certain factors may influence the dosage required to effectively treat a subject, or prevent a subject, suffering from a disease or conditions that benefits from an inhibition of 11β-HSD activity, for example chronic inflammatory diseases, and these factors include, but are not limited to, the severity of the disease or disorder, previous treatments, the general health and/or age of the subject and other diseases present.

Moreover, a *treatment* or *prevention regime* of a subject with a therapeutically effective amount of the compound of the present invention may consist of a single administration, or alternatively comprise a series of applications. For example, the compound of the present invention may be administered at least once a week. However, in another embodiment, the compound may be administered to the subject from about one time per week to about three times daily for a given treatment. The length of the treatment period depends on a variety of factors, such as the severity of the disease, the age of the patient, the concentration and the activity of the compounds of the present invention, or a combination thereof. It will also be appreciated that the effective dosage of the compound used for the treatment or prophylaxis may increase or decrease over the course of a particular treatment or prophylaxis regime. Changes in dosage may result and become apparent by standard diagnostic assays known in the art. In some instances, chronic administration may be required.

As used herein the term *administered contemporaneously* means that two substances are administered to a subject in such a way that they are both biologically active in the subject at the same time. The exact details of the administration will depend on the pharmacokinetics of the two substances in the presence of each other, and can include administering one substance within 24 hours of administration of the other, if the pharmacokinetics is suitable. Designs of suitable dosing regimens are routine for one skilled in the art. In particular embodiments, two substances will be administered substantially simultaneously, i.e. within minutes of each other, or in a single composition that comprises both substances.

To *inhibit* or *suppress* or *reduce* or *downregulate* a function or activity, such 11β-HSD activity, is to reduce the function or activity when compared to otherwise same conditions except for a condition or parameter of interest, or alternatively, as compared to another conditions.

In understanding the scope of the present disclosure, the term *comprising* and its derivatives, as used herein, are intended to be open ended terms that specify the presence of the stated features, elements, components, groups, integers, and/or steps, but do not exclude the presence of other unstated features, elements, components, groups, integers and/or steps. The foregoing also applies to words having similar meanings such as the terms, *including, having* and their derivatives. Finally, terms of degree such as *substantially, about* and *approximately* as used herein mean a reasonable amount of deviation of the modified term such that the end result is not significantly changed. These terms of degree should be construed as including a deviation of at least ±5% of the modified term if this deviation would not negate the meaning of the word it modifies.

Unless otherwise indicated, the terms *a, an,* and *the* as used herein mean one or more that one.

### Compounds of the Invention

A new class of compounds derived from glycyrrhizin has been identified as drugs for the treatment of chronic inflammatory diseases, autoimmune diseases, skin diseases, bone diseases, metabolic diseases, infectious diseases and cancer. The compounds according to the invention may be readily prepared by a person skilled in the art on the basis of his general knowledge. Results show, that glycyrrhizin derivatives, namely compound **1,** inhibit the expression of TNFα and reduce the cytopathic effect associated with virus replication in cell culture. Accordingly, compound **1** and related compounds are a novel class of mechanism-based drugs against chronic inflammatory diseases, autoimmune diseases, skin diseases, bone diseases, metabolic diseases, infectious diseases and cancer.

Accordingly, in one its aspects, the present invention includes a compound selected from a compound of Formula I: 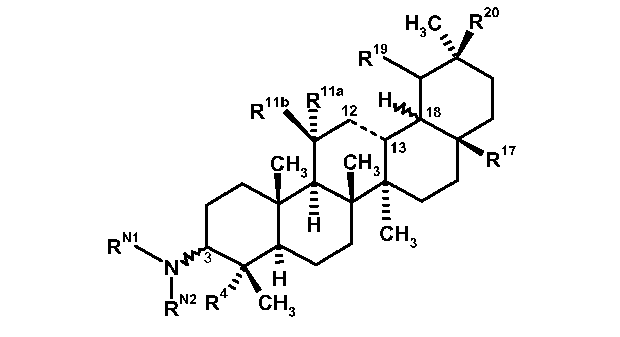 wherein
**R^{N1}** is selected from hydrogen, -**R^{a}**, -O-**R^{a}**, -C(=O)-**R^{a}**; and
**R^{N2}** is selected from -**R^{b}**, -(CH₂)ₙ-NH-**R^{b}**, **-**C(=O)(CH₂)ₙ-N(-O-**R^{a}**)-**R^{b},** -(CH₂)ₙ-N(-O-**R^{a}**)-**R^{b}**, -(CH₂)ₙ-O-**R^{b}**, -(CH₂)ₙ-S-**R^{b}**; and
the configuration at atom **3** and **18** can be independently from one another either R or S; and
**R^{11a}** and **R^{11b}** together are selected from =O, =N-**R^{a}**, =N-O-**R^{a}**; or
**R^{11a}** and **R^{11b}** are independently from one another selected from hydrogen, -O-R^{a}, -0-C(=O)-R^{a}, -NH-R^{a}, methyl, ethyl, ethynyl, fluorine, chlorine, and bromine with the proviso, that **R11^{a}** and **R11^{b}** are not both hydrogen; and
a single or double bond is present at **12-13;**
**R⁴, R¹⁷, R¹⁹** and **R²⁰** are independently from one another selected from hydrogen, methyl, -CH₂-O-**R^{a}**, -CHO, -CO₂-**R^{a}**; and
**R^{a}** is selected from hydrogen, benzhydryl, or from optionally substituted carboxylic acid, C₁₋₆alkyl, C₂₋₆alkenyl, C₂₋₈alkynyl, -CF₃, -(CH₂)ₙ-C₆₋₁₄aryl, -CH=CH-C₆₋₁₄aryl, -C=C-C₆₋₁₄aryl, -(CH₂)ₙ-C₅₋₁₄heteroaryl, -CH=CH-C₅₋₁₄heteroaryl, -C=C-C₅₋₁₄heteroaryl,-(CH₂)ₙ-C₃₋₈Cycloalkyl, -CH=CH-C₃₋₈cycloalkyl and -C=C-C₃₋₈cycloalkyl; and
**R^{b}** is selected from an optionally substituted sugar; and
each **n** independently of one another denotes 0, 1 2, 3, 4, or 5; and optionally in the form of the pharmaceutically effective salts, solvates, prodrugs, tautomers, racemates, enantiomers, diastereomers or mixtures thereof.

One aspect of the invention relates to compounds of general Formula I, wherein the configuration at atom **3** and **18** can be independently from one another either R or S.

One aspect of the invention relates to compounds of general Formula I, wherein **R⁴, R¹⁷** and **R¹⁹** denote methyl.

Another aspect of the invention relates to compounds of general Formula I, wherein **R²⁰** denotes -CO₂-**R^{a}**.

Another aspect of the invention relates to compounds of general Formula I, wherein **R^{11a}** and **R^{11b}** together denote =O; and a double bond is present at 12-13.

Another aspect of the invention relates to compounds of general Formula I, wherein **R^{N2}** is selected from **-R^{b},** -(CH₂)ₙ-S-**R^{b}**, and -C(=O)(CH₂)ₙ-N(-O-**R^{a}**)-**R^{b}**

Another aspect of the invention relates to compounds of general Formula I, wherein **R^{b}** is selected from optionally substituted β-D-glucopyranosiduronic acid, β-D-glucopyranose, α-L-rhamnopyranose, β-D-galactopyranose, β-D-xylopyranose, α-L-arabinofuranose, β-D-quinovopyranose, β-D-apiofuranose, gentibiose, chinovopyranose, fucopyranosyl, β-D-galactopyranosiduronic acid, β-D-ribopyranose, β-D-xylofuranose, β-D-allopyranose, β-D-arabinopyranose and β-D-apiopyranose.

Another aspect of the invention relates to compounds of general Formula I, wherein **R^{N1}** is selected from hydrogen or from optionally substituted methoxy, acetyl and succinyl.

Another aspect of the invention relates to a compound of general Formula I which is selected from the group consisting of
(3S,18R,20S)-11-oxo-3-[(2,3,4,6-tetra-*O*-acetyl-beta-D-glucopyranosyl) methoxamino]-olean-12-en-29-oic acid, diphenylmethyl ester,
(3S,18R,20S)-11-oxo-3-[(2,3,4,6-tetra-*O*-acetyl-beta-D-glucopyranosyl) methoxamino]-olean-12-en-29-oic acid,
(3S,18R,20S)-11-oxo-3-[(beta-D-glucopyranosyl)methoxamino]-olean-12-en-29-oic acid,
(3S,18R,20S)-3-[2-S-(methyl-2,3,4-tri-*O*-acetyl-1-thio-beta-D-glucopyranosyluronate)-ethyl-amino]-11-oxo-olean-12-en-29-oic acid, diphenylmethyl ester,
(3S,18R,20S)-3-[2-S-(methyl-2,3,4-tri-*O*-acetyl-1-thio-beta-D-glucopyranosyluronate)-ethyl-amino]-11-oxo-olean-12-en-29-oic acid,
(3S,18R,20S)-3-[2-S-(methyl-1-thio-beta-D-glucopyranosyluronate)-ethyl-amino]-11-oxo-olean-12-en-29-oic acid,
(3S,18R,20S)-3-[2-(beta-D-glucopyranuronosylth io)-ethyl-amino]-11-oxo-olean-12-en-29-oic acid,
(3S,18R,20S)-3-{*N*-acetyl-N-[2-S-(methyl-2,3,4-tri-*O*-acetyl-1-thio-beta-D-glucopyranosyluronate)-ethyl]-amino}-11-oxo-olean-12-en-29-oic acid, diphenylmethyl ester,
(3S,18R,20S)-3-{*N*-acetyl-*N*-[2-S-(methyl-2,3,4-tri-*O*-acetyl-1-thio-beta-D-glucopyranosyluronate)-ethyl]-amino}-11-oxo-olean-12-en-29-oic acid,
(3S,18R,20S)-3-{*N*-acetyl-*N*-[2-S-(methyl-1-thio-beta-D-glucopyranosyluronate)-ethyl]-amino}-11-oxo-olean-12-en-29-oic acid,
(3S,18R,20S)-3-{*N*-acetyl-*N*-[2-(beta-D-glucopyranuronosylthio)-ethyl]-amino}-11-oxo-olean-12-en-29-oic acid,
(3S,18R,20S)-3-{*N*-(4-methoxy-1,4-dioxobutyl)-N-[2-S-(methyl-2,3,4-tri-*O*-acetyl-1-thio-beta-D-glucopyranosyluronate)-ethyl]-amino}-11-oxo-olean-12-en-29-oic acid, diphenylmethyl ester,
(3S,18R,20S)-3-{*N*-(4-methoxy-1,4-dioxobutyl)-N-[2-S-(methyl-2,3,4-tri-*O*-acetyl-1-thio-beta-D-glucopyranosyluronate)-ethyl]-amino}-11-oxo-olean-12-en-29-oic acid,
(3S,18R,20S)-3-{*N*-(4-methoxy-1,4-dioxobutyl)-N-[2-S-(methyl-1-thio-beta-D-glucopyranosyluronate)-ethyl]-amino}-11-oxo-olean-12-en-29-oic acid and
(3S,18R,20S)-3-{*N*-(4-methoxy-1,4-dioxobutyl)-N-[2-(beta-D-glucopyranurosylthio)-ethyl]-amino}-11-oxo-olean-12-en-29-oicacid

The compounds of the invention may exist in a continuum of solid states ranging from fully amorphous to fully crystalline. The term *amorphous* refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterized by a change of state, typically second order (*glass transition*). The term *crystalline* refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterized by a phase change, typically first order (*melting point*).

The compounds of the invention may also exist in unsolvated and solvated forms. The term *solvate* is used herein to describe a molecular complex comprising the compound of the invention and one or more pharmaceutically acceptable solvent molecules, for example, ethanol. The term *hydrate* is employed when said solvent is water. When the solvent or water is tightly bound, the complex will have a well-defined stoichiometry independent of humidity. When, however, the solvent or water is weakly bound the water/solvent content will be dependent on humidity and drying conditions. In such cases, non-stoichiometry will be the norm.

Herein all references to compounds of Formula I include references to salts, solvates, prodrugs and multi-component complexes thereof.

The compounds of Formula I can be prepared using methods known in the art, for example, 18α- and 18β-glycyrrhetinic acid and their methyl esters may be converted into the corresponding dienones by reaction with 2-iodoxybenzoic acid as per a reported method [8].

The present invention includes radiolabeled forms of the compounds of the invention, for example, compounds of the invention labeled by incorporation within the structure ³H, ¹¹C or ¹⁴C or a radioactive halogen such as ¹²⁵I and ¹⁸F. A radiolabeled compound of the invention may be prepared using standard methods known in the art. For example, tritium may be incorporated into a compound of the invention using standard techniques, for example by hydrogenation of a suitable precursor to a compound of the invention using tritium gas and a catalyst. Alternatively, a compound of the invention containing radioactive iodine may be prepared from the corresponding trialkyltin (suitably trimethyltin) derivative using standard iodination conditions, such as [¹²⁵I] sodium iodide in the presence of chloramine-T in a suitable solvent, such as dimethylformamide. The trialkyltin compound may be prepared from the corresponding non-radioactive halo, suitably iodo, compound using standard palladium-catalyzed stannylation conditions, for example hexamethylditin in the presence of tetrakis (triphenylphosphine) palladium (0) in an inert solvent, such as dioxane, and at elevated temperatures. Further, a compound of the invention containing a radioactive fluorine may be prepared, for example, by reaction of K[¹⁸F]/K222 with a suitable precursor compound, such as a compound of Formula I comprising a suitable leaving group, for example a tosyl group, that may be displaced with the ¹⁸F anion.

### Methods and Compositions

The present invention relates to novel compounds of Formula I, accordingly the present invention includes all uses of these compounds including, for example, in therapeutic and diagnostic applications. The present invention accordingly includes the use of a compound or a combination of compounds of the invention as a medicament or as a diagnostic.

In their ability reduce the cytopathic effect associated with virus replication in cell culture; the compounds of the invention are useful for treating any condition or disease that benefits from a reduction of the cytopathic effect associated with virus replication in cell culture. In an embodiment of the invention, the conditions or diseases that benefit from the reduction of the cytopathic effect associated with virus replication in cell culture are infectious diseases.

Accordingly, the present invention includes a method of treating infectious diseases comprising administering an effective amount of a compound of the invention to a subject in need thereof. The invention also includes a use of a compound of the invention to treat infectious diseases and a use of a compound of the invention to prepare a medicament to infectious diseases. In embodiments of the invention the infectious disease is selected from viral, bacterial or fungal infections.

The present invention also includes a method of treating infectious diseases comprising administering an effective amount of a compound of the invention to a subject in need thereof. Further the invention includes a use of a compound of the invention to treat infectious diseases, as well as a use of a compound of the invention to prepare a medicament to treat these diseases.

In an embodiment of the invention the compound reducing the cytopathic effect associated with viral infections is compound 1. A person skilled in the art would be able to identify compounds of the invention reducing the cytopathic effect associated with viral infections using, for example cellular assays with specific mammalian cell lines and an appropriate virus strain as described in the examples below and in literature.

In their ability to downregulate the expression or activity of TNFα, certain compounds of the invention are useful for treating any condition or disease that benefits from downregulation of TNFα expression. In an embodiment of the invention, the conditions or diseases that benefit from the downregulation of TNFα are chronic inflammatory diseases, autoimmune diseases, skin diseases, bone diseases, metabolic diseases, infectious diseases and cancer.

Accordingly, the present invention includes a method of treating chronic inflammatory diseases, autoimmune diseases, skin diseases, bone diseases, metabolic diseases, infectious diseases and cancer comprising administering an effective amount of a compound of the invention to a subject in need thereof. The invention also includes a use of a compound of the invention to treat chronic inflammatory diseases, autoimmune diseases, skin diseases, bone diseases, metabolic diseases, infectious diseases and cancer and a use of a compound of the invention to prepare a medicament to treat chronic inflammatory diseases, autoimmune diseases, skin diseases, bone diseases, metabolic diseases, infectious diseases and cancer. In embodiments of the invention the chronic inflammatory disease is selected from stomatitis, gingivitis, periodontitis, peri-implantitis and osteoarthritis. In embodiments of the invention the autoimmune disease is selected from rheumatoid arthritis, systemic lupus erythematosus (SLE), Sjögren's syndrome, scleroderma, dermatomyositis, and polymyositis, inflammatory bowel diseases like Crohn's disease and ulcerative colitis. In embodiments of the invention the skin disease is selected from dermatitis, contact dermatitis, allergic dermatitis, atopic dermatitis, psoriasis, eczema, prurigo simplex acuta, prurigo simplex subacuta, prurigo nodularis, alopecia areata, Idiopathic thrombocytopenic purpura, pemphigus vulgaris, actinic keratosis. In embodiments of the invention the bone disease is selected from inflammation and/or immune mediated bone loss, osteoporosis, postmenopausal osteoporosis, Paget's disease, lytic bone metastases, arthritis, juvenile chronic arthritis, adjuvant arthritis, infectious diseases, bone loss by cancer, bone loss by HIV, tooth loss, bone marrow inflammation, synovial inflammation, cartilage and/or bone erosion and/or proteoglycan damage, osteopenie, osteosclerose, osteonecrosis. In embodiments of the invention the metabolic disease or disorder is selected from fasting hyperglycemia, diabetes mellitus, in particular insulin dependent type II diabetes, impaired fasting glucose, impaired glucose tolerance, insulin resistance, high blood pressure, central obesity (also known as visceral adiposity), decreased HDL cholesterol and elevated triglycerides. In embodiments of the invention the infectious disease is selected from viral, bacterial or fungal infections. In embodiments of the invention the cancer is selected from bladder cancer, breast cancer, colorectal cancer, cutaneous melanoma, skin cancer, squamous cell carcinoma of the skin, endometrial cancer, leukemia, lung cancer, non-Hodgkin lymphoma, ovarian cancer, pancreatic cancer, and prostate cancer.

The present invention also includes a method of treating chronic inflammatory diseases, autoimmune diseases, skin diseases, bone diseases, metabolic diseases, infectious diseases and cancer comprising administering an effective amount of a compound of the invention to a subject in need thereof. Further the invention includes a use of a compound of the invention to treat chronic inflammatory diseases, autoimmune diseases, skin diseases, bone diseases, metabolic diseases, infectious diseases and cancer, as well as a use of a compound of the invention to prepare a medicament to treat these diseases.

A person skilled in the art would be able to identify TNFα-downregulating compounds of the invention by contacting one or more cells with a compound of the invention and assaying for the presence of one TNFα and comparing the levels of TNFα in the one or more cells with that of controls. Such methods are known in the art [13,14] and are described in the examples herein below.

The compounds of the invention are suitably formulated into pharmaceutical compositions for administration to human subjects in a biologically compatible form suitable for administration in vivo. Accordingly, in another aspect, the present invention includes a pharmaceutical composition comprising a compound of the invention and a pharmaceutically acceptable carrier or diluent.

The compositions containing the compounds of the invention can be prepared by known methods for the preparation of pharmaceutically acceptable compositions which can be administered to subjects, such that an effective quantity of the active substance is combined in a mixture with a pharmaceutically acceptable vehicle. Suitable vehicles are described in literature [15-17]. On this basis, the compositions include, albeit not exclusively, solutions of the substances in association with one or more pharmaceutically acceptable vehicles or diluents, and contained in buffered solutions with a suitable pH and iso-osmotic with the physiological fluids.

In accordance with the methods of the invention, the described compounds, salts or solvates thereof may be administered to a patient in a variety of forms depending on the selected route of administration, as will be understood by those skilled in the art. The compositions of the invention may be administered, for example, by peroral, parenteral, buccal, sublingual, nasal, rectal, patch, pump or transdermal (topical) administration and the pharmaceutical compositions formulated accordingly. Parenteral administration includes intravenous, intraperitoneal, subcutaneous, intramuscular, transepithelial, nasal, intrapulmonary, intrathecal, rectal and topical modes of administration. Parenteral administration may be by continuous infusion over a selected period of time.

A compound of the invention may be orally administered, for example, with an inert diluent or with an assimilable edible carrier, or it may be enclosed in hard or soft shell gelatin capsules, or it may be compressed into tablets, or it may be incorporated directly with the food of the diet. For oral therapeutic administration, the compound of the invention may be incorporated with excipient and used in the form of ingestible tablets, buccal tablets, troches, capsules, elixirs, suspensions, syrups, wafers, and the like.

A compound of the invention may also be administered parenterally. Solutions of a compound of the invention can be prepared in water suitably mixed with a surfactant such as hydroxypropylcellulose. Dispersions can also be prepared in glycerol, propylene glycol, liquid polyethylene glycols, DMSO and mixtures thereof with or without alcohol, and in oils. Under ordinary conditions of storage and use, these preparations contain a preservative to prevent the growth of microorganisms. A person skilled in the art would know how to prepare suitable formulations.

The pharmaceutical forms suitable for injectable use include sterile aqueous solutions or dispersion and sterile powders for the extemporaneous preparation of sterile injectable solutions or dispersions. In all cases the form must be sterile and must be fluid to the extent that easy syringability exists. Ampoules are convenient unit dosages. In addition, a pharmaceutical form suitable for injectable use includes sterile powders for the direct needle-free injection of the substance into the outer layer of skin in a simple-to-use-device.

Compositions for nasal and pulmonary administration may conveniently be formulated as aerosols, drops, gels and powders. Aerosol formulations typically comprise a solution or fine suspension of the active substance in a physiologically acceptable aqueous or non-aqueous solvent and are usually presented in single or multidose quantities in sterile form in a sealed container, which can take the form of a cartridge or refill for use with an atomizing device.

Alternatively, the sealed container may be a unitary dispensing device such as a single dose inhaler or an aerosol dispenser fitted with a metering valve which is intended for disposal after use. Where the dosage form comprises an aerosol dispenser, it will contain a propellant which can be a compressed gas such as compressed air or an organic propellant such as hydrofluoroalkanes. The aerosol dosage forms can also take the form of a pump-atomizer. Where the dosage form comprises a dry powder inhaler, it can contain a propellant or rely on the force of patient inhalation to entrain powder from the device and subsequently break-up the powder into small aerosol particles

Compositions suitable for buccal or sublingual administration include tablets, lozenges, pastilles, and patches wherein the active ingredient is formulated with a carrier such as sugar, acacia, tragacanth, or gelatin and glycerine. Compositions for rectal administration are conveniently in the form of suppositories containing a conventional suppository base such as cocoa butter.

Compositions for topical administration may include, for example, propylene glycol, isopropyl alcohol, mineral oil and glycerin. Preparations suitable for topical administration include liquid or semi-liquid preparations such as liniments, lotions, applicants, and oil-in-water or water-in-oil emulsions such as creams, ointments or pastes; or solutions or suspensions such as drops. Moreover, suitable preparations for topical administration include patches wherein the ingredient is formulated with carriers such as adhesives, solvents, or polymers. In addition to the aforementioned ingredients, the topical preparations may include one or more additional ingredients such as diluents, buffers, flavoring agents, binders, surface active agents, thickeners, lubricants, preservatives, e.g. methyl hydroxybenzoate (including anti-oxidants), emulsifying agents and the like.

Sustained or direct release compositions can be formulated, e.g. liposomes or those wherein the active compound is protected with differentially degradable coatings, such as by microencapsulation, multiple coatings, etc or those wherein the compound is imbedded into a polymer matrix such as polylactid or the like. It is also possible to freeze-dry the compounds of the invention and use the lyophilisates obtained, for example, for the preparation of products for injection.

The dosage of the compounds of Formula I and/or compositions of the invention can vary depending on many factors such as the pharmacodynamic properties of the compound, the mode of administration, the age, health and weight of the recipient, the nature and extent of the symptoms, the frequency of the treatment and the type of concurrent treatment, if any, and the clearance rate of the compound in the human or animal to be treated. One of skill in the art can determine the appropriate dosage based on the above factors. The compounds of Formula I may be administered initially in a suitable dosage that may be adjusted as required, depending on the clinical response.

The compounds of the invention may be administered to a subject alone or in combination with pharmaceutically acceptable carriers, as noted above, and/or with other pharmaceutically active agents for the treatment of chronic inflammatory diseases, autoimmune diseases, skin diseases, bone diseases, metabolic diseases, infectious diseases and cancer, the proportion of which is determined by the solubility and chemical nature of the compounds, chosen route of administration and standard pharmaceutical practice.

The compounds of Formula I, or salts or solvates thereof, can be used alone or in combination with other agents or therapies that treat inflammatory and autoimmune diseases, for example, but not limited to, non-steroidal anti-inflammatory drugs (NSAIDS), corticosteroids, disease modifying anti-rheumatic drugs (DMARDs), TNFα blockers, interleukin 1 (IL-1) blockers, monoclonal antibodies against B cells, and T cell activation blocker.

The compounds of Formula I, or salts or solvates thereof, can be used alone or in combination with other agents or therapies that treat skin diseases, for example, but not limited to, corticosteroids, immunomodulating agents, retinoids, urea, zinc oxide, panthenol, antibiotics, and antimycotics.

The compounds of Formula I, or salts or solvates thereof, can be used alone or in combination with other agents or therapies that treat metabolic diseases, for example, but not limited to, sulfonylureas, meglitinides, biguanides, thiazolidinediones (TZDs), and α-glucosidase inhibitors.

The compounds of Formula I, or salts or solvates thereof, can be used alone or in combination with other agents or therapies that treat infectious diseases, for example, but not limited to, antibiotics like aminoglycosides, ansamycins, carbacephem, carbapenems, cephalosporins, glycopeptides, macrolides, monobactams, penicillins, polypeptides, quinolones, sulfonamides, and tetracyclines, antimycotics like polyenes, imidazoles, triazoles, allylamines, and echinocandins, and antivirals like cell entry blockers, nucleoside analogues, reverse transcriptase inhibitors, protease inhibitors, and neuraminidase inhibitors.

The compounds of Formula I, or salts or solvates thereof, can be used alone or in combination with other agents or therapies that treat cancer, for example, but not limited to, cytotoxic drugs, kinase inhibitors, antibodies and immunotherapy, selective receptor modulators, non-steroidal anti-inflammatory drugs (NSAIDS), corticosteroids, and enzyme modulators.

While the following Examples illustrate the invention in further detail, it will be appreciated that the invention is not limited to the specific examples.

### Examples

All solvents were purified and dried by standard procedures. Melting points were measured on a Büchi B-545 melting point apparatus or a Kofler hot stage microscope and are uncorrected. Column chromatography was performed on silica gel 60 (230-400 mesh, Merck). Reactions were monitored by TLC on silica gel 60 F₂₅₄ pre-coated glass plates (Merck) or on silica gel 60 F₂₅₄ HPTLC pre-coated glass plates with 2.5 cm concentration zone (Merck); spots were detected by UV light examination or visualized by spraying with anisaldehyde sulfuric acid, molybdophosphoric acid, mixture of molybdophosphoric acid and CeIV ammonium nitrate or ninhydrine and heating. Concentration of solutions was performed at reduced pressure at temperatures <50°C. NMR spectra were recorded at 297 K in CDCl₃, pyridine-d₅, DMSO-d₆ or MeOD with a Bruker AC 200 spectrometer (¹H at 200.13 MHz, ¹³C at 50.31 MHz), a Bruker DPX 300 spectrometer (¹H at 300.13 MHz, ¹³C at 75.47 MHz), a Bruker AC 400 spectrometer (¹H at 400.13 MHz, ¹³C at 100.61 MHz) and with a Bruker DPX 400 spectrometer (¹H at 400.13 MHz, ¹³C at 100.61 MHz) using standard Bruker NMR software. ¹H NMR spectra were referenced to tetramethylsilane. ¹³C NMR spectra were referenced to chloroform (δ 77.00). Infrared spectra were recorded on a BIORAD ATR-FT-IR spectrometer as solutions in DCM or MeOH. Elemental analyses were measured with an EA 1108 CHNS-O from Carlo Erba. Compounds were purified by MPLC (medium pressure liquid chromatography) using preparative silica gel (40-63 mm) columns. HPLC was performed using a Waters 2695 instrument with Merck Chromolith RP18 columns and a gradient of 3 % to 60 % acetonitrile and water containing HCOOH 0.1 % at a flow of 1.0 to 3.0 mL/min. Preparative LC was performed using a Waters instrument with Merck Geminy RP18 columns and a gradient of 3 % to 60 % acetonitrile and water containing HCOOH 0.1 % at a flow of 25 mL/min. The HPLC reported purity is the number generated for the peak area as calculated using the Waters Millennium software with the Maxplot option for the UV maximum of the corresponding peak. Mass spectra were measure in Cl-mode with ammonia as a reagent gas on a thermo scientific.

### Example 1: Compound 1

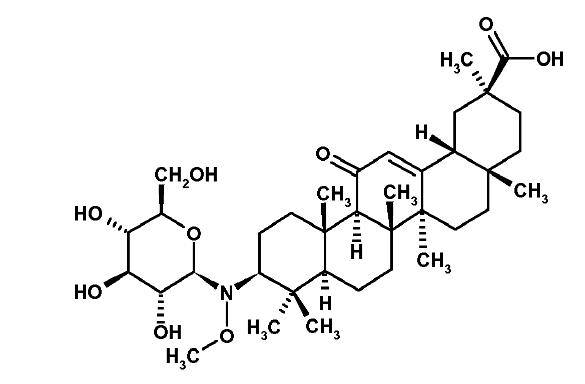

(3S,18R,20S)-11-Oxo-3-[(beta-D-glucopyranosyl)methoxamino]-olean-12-en-29-oic acid

(3S,18R,20S)-3-(*N*-Methoxamino)-11-oxo-olean-12-en-29-oic acid, diphenylmethyl ester (300 mg, 0.45 mmol) and tetraacetyl-D-glucopyranosylbromide (556 mg, 1.311 mmol) were dissolved in dry DCM in oven dried glassware and MS 4A (powdered and activated) and Silver-Zeolite (900 mg) (1 h dried *in vacuo)* were added at RT (room temperature) and the reaction mixture was stirred under TLC monitoring and additional addition of Ag-Zeolite (900 mg, after 3 days) for several days. The reaction mixture was filtered over a short bed of Celite® and evaporated, dissolved in 2.4 mL acetone and was added dropwise to a stirred suspension of Ag₂CO₃ (400 mg) in acetone:water (5.2 mL) and was stirred for 2 h (hours). After 3 h the reaction mixture was filtered over a bed of Celite® and was mostly evaporated and purified by column chromatography (SiO₂, Toluene-EtOAc) to give pure (3S,18R,20S)-11-oxo-3-[(2,3,4,6-tetra-O-acetyl-beta-D-glucopyranosyl)methoxamino]-olean-12-en-29-oic acid, diphenylmethyl ester (125 mg, 27.9 %) as solid foam. The isolated material (100 mg, 0.1 mmol) was dissolved in MeOH and stirred under an atmosphere of H₂ with Pd/C (30 mg) for 3 h upon complete conversion before the reaction atmosphere was changed to argon. The reaction mixture was filtered over a bed of Celite® and purified by column chromatography (SiO₂, CHCl₃:MeOH) to give pure (3S,18R,20S)-11-oxo-3-[(2,3,4,6-tetra-O-acetyl-beta-D-glucopyranosyl)methoxamino]-olean-12-en-29-oic acid (75 mg, 90 %). This peracetate (55 mg, 0.066 mmol) was dissolved in MeOH and 0.1 M NaOMe was added at 0 °C. The reaction mixture was stirred at 0 °C under TLC monitoring for about 2 h. Then the solution was neutralized with anion exchanger. After filtration the filtrate was evaporated and purified by column chromatography (SiO₂, CHCl₃:MeOH) to give (3S,18R,20S)-11-oxo-3-[(beta-D-glucopyranosyl)methoxamino]-olean-12-en-29-oic acid (40 mg, 91 %).

### Physical properties

Optical rotation: [α]_{D}²⁰ = +86.7° (c=0.6, MeOH)

¹H-NMR (400 MHz, MeOD): δ 0.78-0.83 (m, 1H, H5), 0.83 (s, 3H, H28), 0.96 (s, 3H, H23), 0.98 (s, 3H, H24), 0.99-1.11 (m, 2H, H16b, H1b), 1.149 (s, 3H, H26), 1.152 (s, 3H, H29), 1.160 (s, 3H, H25), 1.20-1.28 (m, 1H, H15b), 1.33-1.53 (m, 5H, H6b, H7b, H21 b, H22a, H22b), 1.41 (s, 3H, H27), 1.59-1.77 (m, 3H, H6a, H7a, H19b), 1.77-1.98 (m, 5H, H2a, H2b, H15a, H19a, H21 a), 2.10-2.20 (m, 1H, H16a), 2.20-2.26 (dd, J=13.0Hz, J=4.0Hz, 1H, H18), 2.47 (s, 1H, H9), 2.66-2.74 (m, 1H, H1a), 2.73-2.79 (dm, J=13.2Hz, 1H, H3), 3.16-3.21 (m, 1H, H5#), 3.33-3.31 (m, 2H, H3#,H4#), 3.54 (t, J=8.9Hz, 1H, H2#), 3.70 (dd, J=12.0Hz, J=4.6Hz, 1H, H6b#), 3.81 (dd, J=12.0Hz, J=2.2Hz, 1H, H6a#), 3.86 (d, J=8.9Hz, 1H, H1#), 3.66 (s, 3H, OMe), 5.61 (s, 1H, H12)

¹³C-NMR (100 MHz, CDCl₃): δ 17.0 (q, C25), 18.8 (q, C24), 18.8 (t, C7), 19.3 (q, C26), 21.8 (t, C2), 23.8 (q, C27), 27.4 (t, C16), 27.6 (t, C15), 29.2 (q, C28), 28.9 (q, C29), 29.2 (q, C23), 32.2 (t, C21), 33.0 (s, C17), 33.9 (t, C6), 38.2 (s, C10), 39.1 (t, C22), 39.8 (s, C4), 41.6 (t, C1), 42.7 (t, C19), 44.6 (s, C20), 45.2 (s, C14), 46.8 (s, C8), 49.9 (d, C18), 57.1 (d, C5), 62.7 (t, C#6), 63.2 (d, C9), 63.8 (d, OCH3), 71.0 (d, C#4), 72.0 (d, C#2), 74.9 (d, C3), 79.5 (d, C#3), 79.7 (d, C#5), 100.1 (d, C#1), 128.9 (d, C12), 173.0 (s, C13), 181.3 (s, C30), 202.8 (s, C11)

### Example 2: Compound 2

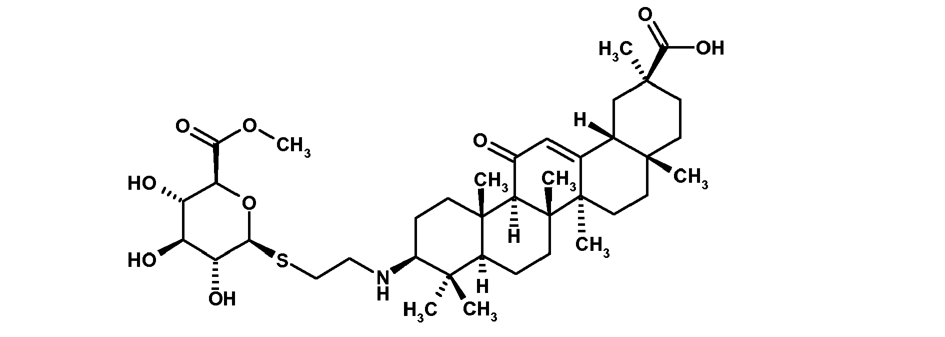

(3S,18R,20S)-3-[2-S-(Methyl-1-thio-beta-D-glucopyranosyluronate)-ethyl-amino]-11-oxo-olean-12-en-29-oic acid

A solution of (3S,18R,20S)-3-amino-11-oxo-olean-12-en-29-oic acid, diphenylmethyl ester (1.32 g, 2.08 mmol) and Hünig's base (1.34 g, 10.4 mmol) in DMF (20 %) solution was purged with Ar for several minutes at RT before 2,3,4-tri-O-acetyl-S-(2-iodoethyl)-1-thio-beta-D-glucopyranuric acid, methylester (2.09 g, 4.15 mmol) was added and the reaction mixture was stirred at 45 °C external temperature under HPLC monitoring. Upon complete conversion the reaction mixture, diluted with EtOAc, washed with NaHCO₃ twice, several times with water, was dried over Na₂SO₄ evaporated and coevaporated with toluene. The crude material was purified by column chromatography (SiO₂, CHCl₃:MeOH 40:1+0.1 % AcOH to 20:1 + 0.1 % AcOH) to give pure (3S,18R,20S)-3-[2-S-(methyl-2,3,4-tri-*O*-acetyl-1-thio-beta-D-glucopyranosyluronate)-ethyl-amino]-11-oxo-olean-12-en-29-oic acid, diphenylmethyl ester (1.5 g, 71.4 %) as solid white foam. This material (300 mg, 0.296 mmol) dissolved in dry DCM anisole (0.3 mL),was added and the reaction mixture was cooled to 0 °C before TFA (1 mL) was added dropwise and the reaction mixture was allowed to come to RT and was stirred under TLC (CHCl₃:MeOH 15:1) monitoring. After 3 h all starting material was consumed and the reaction was diluted with EtOAc (50 mL) and neutralized by addition of saturated NaHCO₃ until the aqueous layer stayed slightly basic. The aqueous layer was acidified by addition of AcOH and extracted with EtOAc three times. The organic layers were washed with brine, dried over Na₂SO₄ and evaporated. The crude material was purified by column chromatography (SiO₂ DCM:MeOH 20:1+0.1% AcOH) to give pure (3S,18R,20S)-3-[2-S-(methyl-2,3,4-tri-*O*-acetyl-1-thio-beta-D-glucopyranosyluronate)-ethyl-amino]-11-oxo-olean-12-en-29-oic acid in 260 mg (96.8 %) yield. This material was dissolved in dry MeOH and 0.1 M NaOMe was added at 0 °C and the pH was adjusted to about 10.5. After 3 h all starting material was converted to the corresponding methylester and the reaction mixture was neutralized (pH 6) by addition of freshly washed ion exchange resin. Filtration, washing and evaporation gave (3S,18R,20S)-3-[2-S-(methyl-1-thio-beta-D-glucopyranosyluronate)-ethyl-amino]-11-oxo-olean-12-en-29-oic acid (200 mg, 89.4 %) in pure form.

### Physical properties

Optical rotation: [α]_{D}²⁰ = +97.90 (c=9.8, MeOH)

MS: calc [M+1]: 720.4145; found [M+1]: 720.4105; dev.: -5 ppm

¹H-NMR (300 MHz, MeOD): δ 0.83 (s, 3H, H28), 0.87-0.95 (m, 1H, H5), 0.93 (s, 3H, H24), 0.97-1.09 (m, 2H, H1b, H16b), 1.12 (s, 6H, H23, H29), 1.16 (s, 3H, H26), 1.17 (s, 3H, H25), 1.19-1.40 (m, 4H, H7b, H15b, H21 b, H22b), 1.43 (s, 3H, H27), 1.44-1.58 (m, 2H, H6b, H22a), 1.58-1.78 (m, 4H,H2b, H6a, H7a, H19b), 1.79-2.05 (m, 4H, H2a, H15a, H19a, H21 a), 2.08-2.23 (m, 1H, H16a), 2.25-2.34 (m, 1H, H18), 2.49 (s, 1H, H9), 2.65-2.76 (m, 1H, H3), 2.79-2.89 (m, 1H, H1a), 2.88-3.13 (m, 2H, S-CH2), 3.08-3.30 (m, 2H, NCH2), 3.22-3.34 (m, 1H, H2#), 3.40 (t, J=8.6Hz, 1H, H3#), 3.53 (t, J=9.0Hz, 1H, H4#), 3.78 (s, 3H, OMe), 3.92 (d, J=9.5Hz, 1H, H5#), 4.54 (d, J=9.2Hz, 1H, H1#), 5.67 (s, 1H, H12)

¹³C-NMR (75 MHz, MeOD): 16.7 (q, C24), 16.8 (q, C25), 18.6 (t, C7), 19.3 (q, C26), 22.2 (t, C2), 23.8 (q, C27), 27.5 (t, C15), 27.7 (t, C16), 28.2 (t, S-CH2), 28.3 (q, C23), 29.3 (q, C28), 29.3 (q, C29), 32.7 (t, C21), 33 (s, C17), 33.7 (t, C6), 38.2 (s, C10), 38.8 (s, C4), 39.4 (t, C22), 39.8 (t, C1), 43.3 (t, C19), 44.7 (s, C20), 45.8 (s, C14), 46.6 (s, C8), 47.6 (t, N-CH2), 50 (d, C18), 53 (q, OMe), 56.5 (d, C5), 62.8 (d, C9), 67.5 (d, C3), 72.9 (d, C4#), 73.5 (d, C2#), 78.7 (d, C3#), 80.1 (d, C5#), 87.7 (d, C1#), 128.9 (d, C12), 171.1 (s, COOMe), 173.6 (s, C13), 183 (s, C30), 202.2 (s, C11)

### Example 3: Compound 3

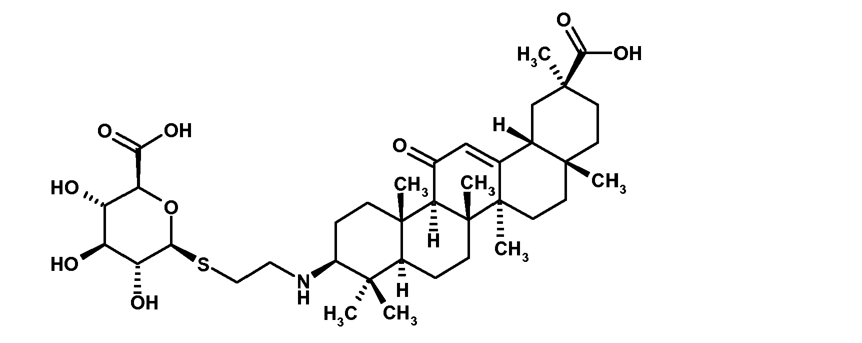

(3S,18R,20S)-3-[2-(β-D-glucopyranuronosylthio)-ethyl-amino]-11-oxo-olean-12-en-29-oic acid

(3S,18R,20S)-3-[2-S-(Methyl-1-thio-beta-D-glucopyranosyluronate)-ethyl-amino]-11-oxo-olean-12-en-29-oic acid (200 mg, 0.28 mmol) was dissolved in little dry MeOH and 0.2 N NaOH in MeOH (6 mL) was added at 0 °C. The reaction mixture was stirred under TLC monitoring at RT. After about 3.5 h the reaction mixture was neutralized to pH 6-7 with freshly washed ion exchange resin and was filtered, washed and evaporated to give 200 mg (97 %) (3S,18R,20S)-3-[2-(β-D-glucopyranuronosyl-thio)-ethyl-amino]-11-oxo-olean-12-en-29-oic acid.

### Physical properties

Optical rotation: [α]_{D}²⁰ = +119.5° (c=1.0, MeOH)

MS: calc [M+1]: 706.3989; found [M+1]: 706.3900; dev.: -12.60 ppm

¹H-NMR (400 MHz, MeOD): δ 0.74 (s, 3H, H28), 0.84-0.91 (m, 1H, H5), 0.91 (s, 3H, H24), 0.91-0.96 (m, 1H, H16b), 0.99-1.05 (m, 1H, 1b), 1.02 (s, 3H, H29), 1.07 (s, 3H, H26), 1.09 (s, 3H, H25), 1.10 (s, 3H, H23), 1.12-1.31 (m, 3H, H15b, H21 b, H22b), 1.34 (s, 3H, H27), 1.34-1.45 (m, 3H, H6b, H7b, H22a), 1.47-1.63 (m, 2H, H7a, H19b), 1.63-1.83 (m, 5H, H2a, H2b, H6a, H15a, H19a), 1.82-1.89 (m, 1H, H21a), 2.07 (td, J=13.5Hz, J=4.3Hz, 1H, H16a), 2.20 (dd, J=13.1Hz, J=4.7Hz, 1H, H18), 2.42 (s, 1H, H9), 2.74-2.82 (m, 1H, H1 a), 2.90 (dd, J=11.8Hz, J=4.7Hz, 1H, H3), 2.94-3.08 (m, 2H, S-CH2), 3.11-3.17 (m, 1H, H5#), 3.28-3.39 (m, 4H, NCH2, H3#,H4#), 3.52-3.58 (m, 1H, H2#), 4.50 (d, J=9.8Hz, 1H, H1#), 5.59 (s, 1H, H12)

¹³C-NMR (100 MHz, MeOD): 16.7 (q, C24, C25), 18.5 (t, C7), 19.3 (q, C26), 20.9 (t, C2), 23.8 (q, C27), 27.2 (t, SCH2), 27.5 (t, C16), 27.6 (t, C15), 28.2 (q, C23), 29.3 (q, C28, C29), 32.6 (t, C6), 33 (s, C17), 33.6 (t, C21), 38.1 (s, C10), 38.8 (s, C4), 39.4 (t, C1), 39.4 (t, C22), 43.3 (t, C19), 44.7 (s, C20), 45.2 (t, NCH2), 45.8, 46.6 (2xs, C8, C14), 50 (d, C18), 56.3 (d, C5), 62.6 (d, C9), 65.8 (d, C3), 73.4 (d, C4#), 74.2 (d, C2#), 78.8 (d, C5#), 79.2 (d, C3#), 86.8 (d, C1#), 128.8 (d, C12), 173.7 (s, C13), 176.2 (s, C6#), 183.1 (s, C30), 202.1 (s, C11)

### Example 4: Compound 4

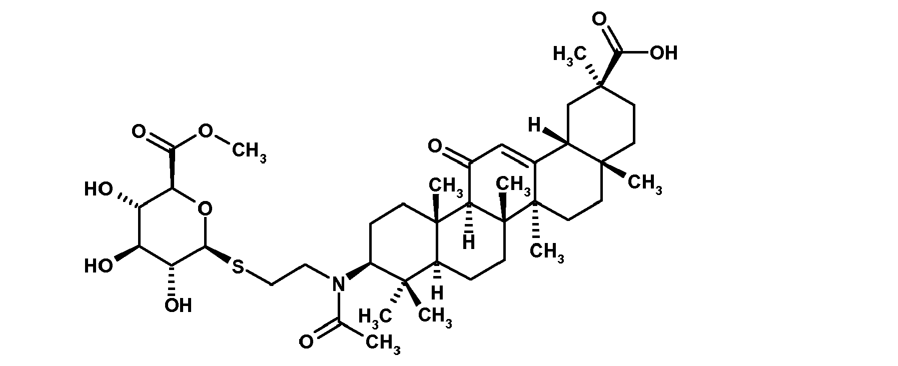

(3S,18R,20S)-3-{*N*-Acetyl-*N*-[2-S-(methyl-1-thio-beta-D-glucopyranosyluronate)-ethyl]-amino}-11-oxo-olean-12-en-29-oic acid

A solution of (3S,18R,20S)-3-amino-11-oxo-olean-12-en-29-oic acid, diphenylmethyl ester (1.32 g, 2.08 mmol) and Hünig's base (1.34 g, 10.4 mmol) in DMF (20 %) solution was purged with Ar for several minutes at RT before 2,3,4-tri-O-acetyl-S-(2-iodoethyl)-1-thio-beta-D-glucopyranuric acid, methylester (2.09 g, 4.15 mmol) was added and the reaction mixture was stirred at 45 °C external temperature under HPLC monitoring. Upon complete conversion the reaction mixture diluted with EtOAc, washed with NaHCO₃ two times, several times with water, was dried over Na₂SO₄ evaporated and coevaporated with toluene. The crude material was purified by column chromatography (SiO₂, CHCl₃:MeOH 40:1+0.1 % AcOH to 20:1 + 0.1 % AcOH) to give pure (3S,18R,20S)-3-[2-*S*-(methyl-2,3,4-tri-*O*-acetyl-1-thio-beta-D-glucopyranosyluronate)-ethyl-amino]-11-oxo-olean-12-en-29-oic acid, diphenylmethyl ester (1.5 g, 71.4 %) as solid white foam. This material (420 mg, 0.415 mmol) dissolved in DCM was chilled to 0 °C before TEA (294 mg, 2.9 mmol) first and then Ac₂O (0.21 g, 2.07 mmol) was added and the reaction mixture was stirred at 0 °C for 15 min and allowed to come to RT afterwards. After 2.5 h all starting material was consumed according to TLC (Hex:EtOAc 1:2). After 4 h at RT the reaction mixture was diluted with EtOAc and washed with diluted AcOH twice, twice with NaHCO₃, once with brine, dried over Na₂SO₄ and evaporated to give crude material which was purified by column chromatography (SiO0₂: Tol:EtOAc) to give (3S,18R,20S)-3-{*N*-acetyl-*N*-[2-S-(methyl-2,3,4-tri-*O*-acetyl-1-thio-beta-D-glucopyranosyluronate)-ethyl]-amino}-11-oxo-olean-12-en-29-oic acid, diphenylmethyl ester (364 mg, 83 %) in pure form as white solid foam. This material (288 mg, 0.273 mmol) was dissolved in dry DCM and chilled to 0 °C before first anisole (0.3 mL) and then portion wise TFA (1 mL) was added and the reaction mixture was stirred at 0°C for 3 h at RT for several more h. Upon completion of reaction (TLC, CHCl₃:MeOH 15:1) the reaction mixture was treated with 10 mL of saturated NaHCO₃ for 20 min (minutes) under stirring. The aqueous layer was acidified by addition of several drops of acetic acid (pH 4), the organic layer was separated and the aqueous layer was extracted with DCM. The combined organic layers were washed with water twice, once with brine, dried over Na₂SO₄ and evaporated. The residue was purified by column chromatography (SiO₂: CHCl₃ to CHCl₃: MeOH 30:1) to give pure give (3S,18R,20S)-3-{*N*-acetyl-*N*-[2-S-(methyl-2,3,4-tri-*O*-acetyl-1-thio-beta-D-glucopyranosyluronate)-ethyl]-amino}-11-oxo-olean-12-en-29-oic acid as white solid (235 mg, 96.9 %). The isolated material was dissolved in dry MeOH and 0.1 M NaOMe was added at 0 °C (pH about 10.5) and the reaction was allowed to come to RT and was stirred under HPLC monitoring. Upon completion of reaction, the reaction mixture was neutralized with ion exchange resin to pH 4-5, filtered and washed. Evaporation gave pure (3S,18R,20S)-3-{*N*-acetyl-*N*-[2-S-(methyl-1-thio-beta-D-glucopyranosyluronate)-ethyl]-amino}-11-oxo-olean-12-en-29-oic acid (160 mg, 93 %) as white solid.

### Physical properties

Optical rotation: [α]_{D}²⁰ = +39.9° (c=1.1, CHCl₃:MeOH 3:1)

MS: calc [M+1]: 762.4251; found [M+1]: 762.3536; dev.: -94 ppm

### Example 5: Compound 5

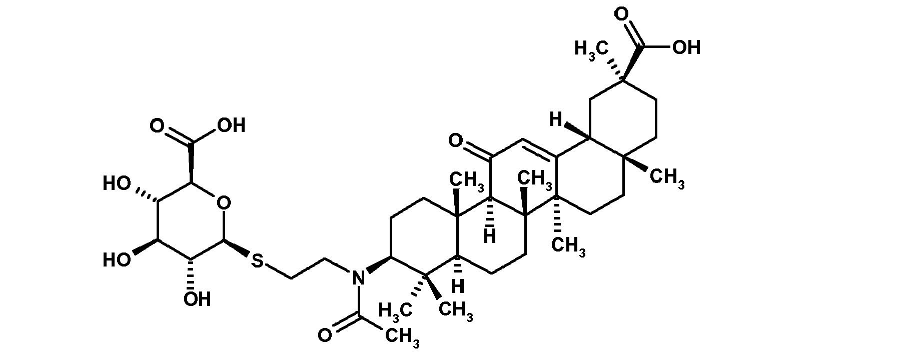

(3S,18R,20S)-3-{*N*-Acetyl-*N*-[2-(beta-D-glucopyranuronosylthio)-ethyl]-amino}-11-oxo-olean-12-en-29-oic acid

(3S,18R,20S)-3-{*N*-Acetyl-*N*-[2-S-(methyl-1-thio-beta-D-glucopyranosyluronate)-ethyl]-amino}-11-oxo-olean-12-en-29-oic acid (70 mg, 0.092 mmol) was dissolved in 0.2 M NaOH in dry MeOH at RT and was stirred at RT under HPLC and TLC monitoring (EtOAc:MeOH 3:2 + AcOH). Upon complete conversion of the starting material the reaction mixture was neutralized by ion exchange resin (pH about 4), filtered, washed and evaporated to give pure (3S,18R,20S)-3-{*N*-acetyl-*N*-[2-(beta-D-glucopyranuronosylthio)-ethyl]-amino}-11-oxo-olean-12-en-29-oic acid (65 mg, 95 %).

### Physical properties

Optical rotation: [α]_{D}²⁰ = +38.0° (c=9.7, MeOH:CHCl₃ 5:1)

MS: calc [M+1]: 748.4094; found [M+1]: 748.3978; dev.: -15 ppm

### Example 6: Compound 6

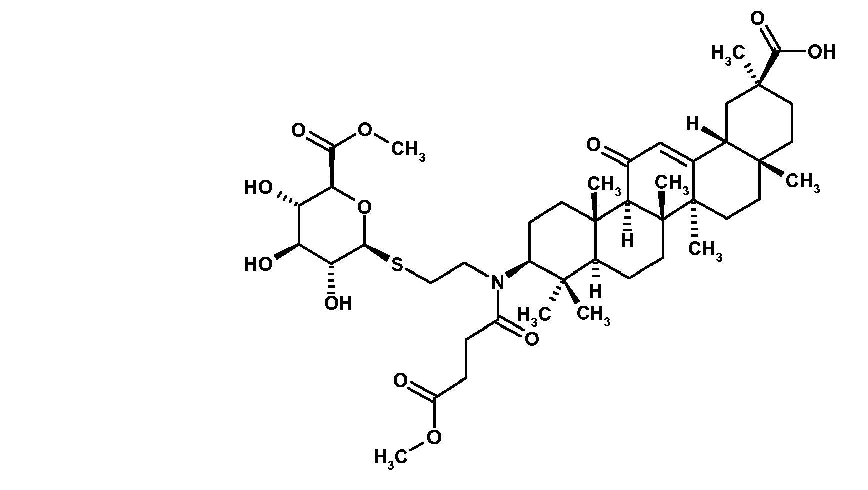

(3S,18R,20S)-3-{*N*-(4-Methoxy-1,4-dioxobutyl)-*N*-[2-*S*-(methyl-1-thio-beta-D-glucopyranosyluronate)-ethyl]-amino}-11-oxo-olean-12-en-29-oic acid

A solution of (3S,18R,20S)-3-amino-11-oxo-olean-12-en-29-oic acid, diphenylmethyl ester (1.32 g, 2.08 mmol) and Hünig's base (1.34 g, 10.4 mmol) in DMF (20 %) solution was purged with Ar for several min at RT before 2,3,4-tri-O-acetyl-S-(2-iodoethyl)-1-thio-beta-D-glucopyranuric acid, methylester (2.09 g, 4.15 mmol) was added and the reaction mixture was stirred at 45 °C external temperature under HPLC monitoring. Upon complete conversion the reaction mixture diluted with EtOAc, washed with NaHCO₃ twice, several times with water, was dried over Na₂SO₄ evaporated and coevaporated with toluene. The crude material was purified by column chromatography (SiO₂, CHCl₃:MeOH 40:1+0.1% AcOH to 20:1 + 0.1% AcOH) to give pure (3S,18R,20S)-3-[2-*S*-(methyl-2,3,4-tri-*O*-acetyl-1-thio-beta-D-glucopyranosyluronate)-ethyl-amino]-11-oxo-olean-12-en-29-oic acid, diphenylmethyl ester (1.5 g, 71.4 %) as solid white foam. This material (350 mg, 0.346 mmol) was dissolved in dry DCM (10 mL), DIPEA (0.28 mL, 1.73 mmol) was added and the reaction mixture was chilled to 0 °C. Succinic acid monochloride monoester (0.156 mg, 1.037 mmol) was added at 0 °C within 5 min and the reaction mixture was stirred at 0 °C for 1.5 h. According to HPLC all starting material was converted to the target compound. MeOH (2 mL) was added and the reaction mixture was allowed to come to RT. NH₄Cl was added followed by EtOAc (40 mL). The phases were separated, the aqueous layer was extracted with EtOAc and the combined organic layers were washed with NH₄Cl and brine, dried over Na₂SO₄ and evaporated. The crude material was purified by column chromatography (SiO₂, Hex:EtOAc) to give pure (3S,18R,20S)-3-{*N*-(4-methoxy-1,4-dioxobutyl)-*N*-[2-S-(methyl-2,3,4-tri-*O*-acetyl-1-thio-beta-D-glucopyranosyluronate)-ethyl]-amino}-11-oxo-olean-12-en-29-oic acid, diphenylmethyl ester as white solid foam (379 mg, 97.3 %) after drying *in vacuo* overnight. This material (350 mg, 0.311 mmol) was dissolved in dry DCM and chilled to 0 °C before first anisole (0.34 mL) and then portion wise TFA (1 mL) was added and the reaction mixture was stirred at 0 °C for 3 h under TLC monitoring Upon complete conversion (TLC, CHCl₃:MeOH 15:1) the reaction mixture was diluted with EtOAc and washed with water and brine excessively. The aqueous layers were extracted with EtOAc, the combined organic layers were washed with brine, dried over Na₂SO₄ and evaporated to give crude material, which was purified by column chromatography (SiO₂ Hex:EtOAc) to give (3S,18R,20S)-3-{*N-*(4-methoxy-1,4-dioxobutyl)-*N*-[2-S-(methyl-2,3,4-tri-*O*-acetyl-1-thio-beta-D-glucopyranosyluronate)-ethyl]-amino}-11-oxo-olean-12-en-29-oic acid in pure form. This material (70 mg, 0.073 mmol) was dissolved in dry MeOH and 0.1 M NaOMe was added at 0°C. The pH was adjusted to pH 10.0. The reaction mixture was stirred at 0 °C under TLC (EE:Hex 2:1, DCM:MeOH 10:1+AcOH) and HPLC monitoring for several h. Upon complete conversion the reaction mixture was neutralized with ion exchange resin (pH 4-5) filtered, evaporated and purified by column chromatography (SiO₂: DCM:MeOH 20:1+0.1% AcOH) to give after coevaporation from toluene and drying pure (3S,18R,20S)-3-{*N*-(4-methoxy-1,4-dioxobutyl)-*N*-[2-S-(methyl-1-thio-beta-D-glucopyranosyluronate)-ethyl]-amino}-11-oxo-olean-12-en-29-oic acid (60 mg, 98.7 %) as white solid.

### Physical properties

Optical rotation: [α]_{D}²⁰ = +37.4° (c=0.5, CHCl₃:MeOH 5:1)

MS: calc [M+1]: 834.4462; found [M+1]: 834.4849; dev.: 46 ppm

### Example 7: Compound 7

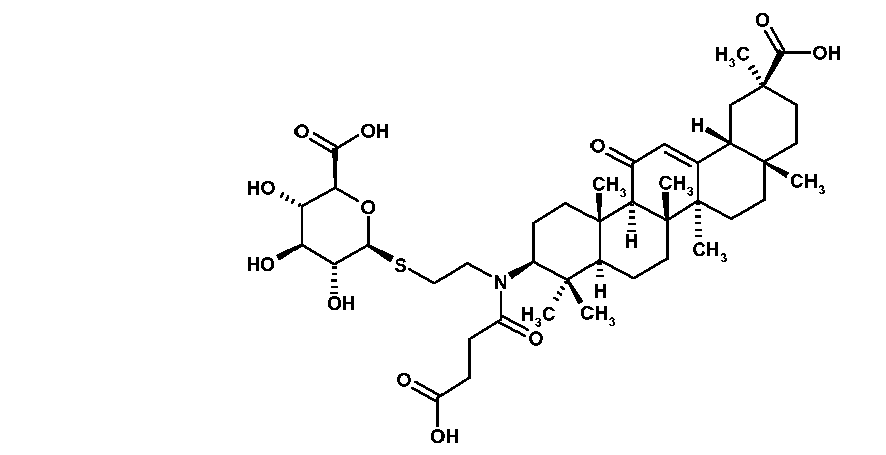

(3S,18R,20S)-3-{*N*-(4-Methoxy-1,4-dioxobutyl)-*N*-[2-(beta-D-glucopyranurosylthio)-ethyl]-amino}-11-oxo-olean-12-en-29-oic acid

(3S,18R,20S)-3-{*N*-(4-Methoxy-1,4-dioxobutyl)-N-[2-S-(methyl-1-thio-beta-D-glucopyranosyluronate)-ethyl]-amino}-11-oxo-olean-12-en-29-oic acid was dissolved in 0.2 N NaOH in dry MeOH at RT, 0.4 mL of HPLC grade water was added and the reaction mixture was stirred at RT overnight. According to HPLC complete conversion from starting material to target compound was achieved. The reaction mixture was neutralized with ion exchange resin and pH was adjusted to about 8. The mixture was filtered, washed and evaporated, taken up in mixture of dioxane:water 1:1 (3 mL) and lyophilized to yield pure (3S,18R,20S)-3-{*N*-(4-methoxy-1,4-dioxobutyl)-*N*-[2-(beta-D-glucopyranurosylthio)-ethyl]-amino}-11-oxo-olean-12-en-29-oic acid (62 mg, 98.8 %) as white lyophilisate.

### Physical properties

Optical rotation: [α]_{D}²⁰ = +33.9° (c=0.6, MeOH)

MS: calc [M+1]: 806.4149; found [M+1]: 806.4572; dev.: +52.5ppm

### Example 8: Effects on the Replication of H3N2 Influenza A Virus

### Experimentals:

Growth medium (DMEM/Ham's F-12 (1:1), PAA Cat. No. E15-012; 10 % FBS, PAA Cat. No. A15-101; 2 mM L-Glutamine, PAA Cat. No. M11-006; 1 % antibiotics, PAA Cat. No. P11-002) of >70 % confluent MDCK cells was removed, the cells were washed once with 10 mL PBS (PAA Cat. No. H15-002) and detached from the culture flask surface by trypsination using 5 mL trypsin-EDTA (PAA Cat. No. L11-660). Detached cells were resuspended with 5 mL growth medium, counted, and seeded at 2x10E4 cells/well in a 96-well plate in growth medium 16 h prior to infection.

A 5 mM stock solution of glycyrrhizin (Fluka, Cat. No. 50531) was prepared in infection medium (DMEM/Ham's F-12 (1:1) containing 5 µg/mL Trypsin, Sigma, Cat. No. T4549, 0.5 % DMSO, Sigma, Cat. No. D4540, and 1 % antibiotics). The virus stock (MDCK stock 080929, titer 5x10*6TCID50/ml) was diluted in infection medium 1:500 to achieve a theoretical MOI (multiplicity of infection) of 0.05. Prior infection, the growth medium was removed and the cells washed once with 200 µL PBS. After removal of PBS, to the untreated wells 50 µL infection medium plus 50 µL virus dilution were added, to the treated wells 20 µL infection medium, 30 µL glycyrrhizin stock (1.5 mM glycyrrhizin final) and 50 µL virus dilution were added. In the wells used for cytotoxicity determination (uninfected cells), the virus dilution was replaced by infection medium. Each treatment was conducted in replicates.

After incubation at 37 °C, 5 % CO₂ for 48 h, cell viability was determined using CellTiter-Glo luminescent cell viability assay (Promega, Cat. No. G7571). The supernatant was removed carefully and 65 µL of the reconstituted reagent were added to each well and incubated for 15 min at RT under gently shaking. Then, 60 µL of the solution was transferred to a white plate and luminescence was measured.

### Results:

It was found that the cell viability of uninfected 1.5 mM glycyrrhizin treated cells is not significantly different from the viability of uninfected untreated cells. Treatment of infected cells with 1.5 mM glycyrrhizin results on average in a reduction of the CPE (cytopathic effect) of 57 % (+/- 15.7 %; n=20). Cell viability of untreated infected cells in a similar setting is 7.6 % (+/- 2.6; n=4).

The table below shows the average reduction of the CPE and the cell viability upon treatment with the current compounds:

| **Compound** | **Concentration** µ**M** | **Viability %** | **cpe reduction %** |
|---|---|---|---|
| Glycyrrhizin | 25 | 100.9 | 4.1 |
| | 250 | 105.3 | 8.1 |
| | 1500 | 101.7 | 47.8 |
| Glycyrrhetinic acid | 25 | 0.2 | |
| | 250 | 0.0 | |
| 2 | 25 | 114.6 | -1.3 |
| | 250 | 101.2 | 25.3 |
| 3 | 25 | 103.5 | -4.2 |
| | 250 | 105.5 | 87.0 |
| 4 | 25 | 104.4 | 6.2 |
| | 250 | 99.4 | 92.3 |
| 5 | 25 | 106.6 | -1.4 |
| | 250 | 93.5 | 58.5 |
| 6 | 25 | 107.3 | -0.5 |
| | 250 | 106.3 | 87.3 |
| 7 | 25 | 104.3 | 3.6 |
| | 250 | 106.1 | 12.1 |

### Reference List:

[1] R.A.Isbrucker, G.A.Burdock, Regul Toxicol Pharmacol 46 (2006) 167-192.
[2] N.Nassiri Asl, H.Hosseinzadeh, Journal of Medicinal Plants 6 (2007) 1-12.
[3] M.Nassiri Asl, H.Hosseinzadeh, Phytotherapy Research 22 (2008) 709-724.
[4] F.Andersen, International Journal of Toxicology 26 (2007) 79-112.
[5] J.Rios et al., Studies in Natural Product Chemistry 22 (2000) 93-143.
[6] Y.H.Zhang et al., Immunology. 79 (1993) 528-534.
[7] Y.H.Zhang et al., Cell. Immunol. 162 (1995) 97-104.
[8] K.C.Nicolaou et al., JACS 124 (2002) 2245-2258.
[9] R.A.Schweizer et al., Mol. Cell Endocrinol. 212 (2003) 41-49.
[10] X.Su et al., J. Steroid Biochem. Mol. Biol. 104 (2007) 312-320.
[11] S.Diederich et al., Eur. J. Endocrinol. 142 (2000) 200-207.
[12] S.A.Latif et al Mol. Cell Endocrinol. 243 (2005) 43-50.
[13] A.Ukil et al., J Immunol. 175 (2005) 1161-1169.
[14] N.Abe, T.Ebina, N.Ishida, Microbiol. Immunol. 26 (1982) 535-539.
[15] R.C.Rowe, P.J.Sheskey, S.C.Owen, Handbook of Pharmaceutical Excipients, 5 ed., Pharmaceutical Press, London, Chicago, 2006.
[16] United States Pharmacopeia, USP NF 2008 (United States Pharmacopeia/National Formulary), 2008.
[17] European Directorate for the Quality of Medicines & HealthCare (EDQM), European Pharmacopoeia, 2008.
[18] A.Odermatt et al., J. Biol. Chem. 274 (1999) 28762-28770.
[19] G.Escher et al., J. Exp. Med. 186 (1997) 189-198.
[20] D. Schuster et al., J. Med. Chem. 49 (2006) 3454-3466.
[21] X. Su et al., Bioorg. & Med. Chem. 12 (2004) 4439-4457.
[22] C. Brieskorn et al., Archiv der 303 (1970) 901-4.
[23] A. Ech-Chahad et al. Tetrahedron Letters 46 (2005) 5113-5115.
[24] W. J. Boyle et al., Nature 423 (2003) 337-342.
[25] T. Suda et al., Bone 17 (1995)

## Claims

1. Compounds of general structural Formula I, 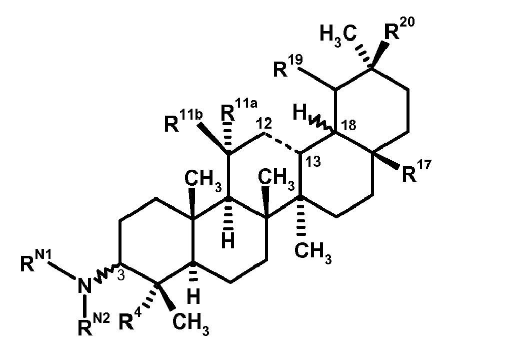 wherein
**R^{N1}** is selected from hydrogen, **-R^{a},** -O-**R^{a}**, -C(=O)-**R^{a}**; and
**R^{N2}** is selected from -**R^{b}**, -(CH₂)ₙ-NH-**R^{b}**, -C(=O)(CH₂)ₙ-N(-O-**R^{a}**)-**R^{b}**, -(CH₂)ₙ-N(-O-**R^{a}**)-**R^{b}**, -(CH₂)ₙ-O-**R^{b}**, -(CH₂)ₙ-S-**R^{b}**; and
**R^{11a}** and **R^{11b}** together are selected from =O, =N-**R^{a}**, =N-O-**R^{a}**; or
**R^{11a}** and **R^{11b}** are independently from one another selected from hydrogen, -O-R^{a}, -0-C(=O)-R^{a}, -NH-R^{a}, methyl, ethyl, ethynyl, fluorine, chlorine, and bromine with the proviso, that **R^{11a}** and **R^{11b}** are not both hydrogen; and
a single or double bond is present at **12-13;**
**R⁴, R¹⁷, R¹⁹** and **R²⁰** are independently from one another selected from hydrogen, methyl, -CH₂-O-**R^{a}**, -CHO, -CO₂-**R^{a}**; and
**R^{a}** is selected from hydrogen, benzhydryl, or from optionally substituted carboxylic acid, C₁₋₆alkyl, C₂₋₆alkenyl, C₂₋₈alkynyl, -CF₃, -(CH₂)ₙ-C₆₋₁₄aryl, -CH=CH-C₆₋₁₄aryl, -C≡C-C₆₋₁₄aryl, -(CH₂)ₙ-C₅₋₁₄heteroaryl, -CH=CH-C₅₋₁₄heteroaryl, -C≡C-C₅₋₁₄heteroaryl,-(CH₂)ₙ-C₃₋₈cydoalkyl, -CH=CH-C₃₋₈cycloalkyl and -C≡C-C₃-₈cycloalkyl; and
**R^{b}** is selected from an optionally substituted sugar; and
each n independently of one another denotes 0, 1 2, 3, 4, or 5; and
optionally in the form of the pharmaceutically effective salts, solvates, prodrugs, tautomers, racemates, enantiomers, diastereomers or mixtures thereof.

2. Compounds according to claim 1, wherein
**R⁴, R¹⁷** and **R¹⁹** denote methyl.

3. Compounds according to claim 1 or 2, wherein **R²⁰** denotes -CO₂-**R^{a}**

4. Compounds according to one of claims 1 to 3, wherein
**R^{11a}** and **R^{11b}** together denote =O; and a double bond is present at 12-13.

5. Compounds according to one of claims 1 to 4, wherein **R^{N2}** is selected from **-R^{b},** -(CH₂)ₙ-S-**R^{b}**, and -C(=O)(CH₂)ₙ-N(-O-**R^{a}**)-**R^{b}**

6. Compounds according to claim 5, wherein **R^{b}** is selected from optionally substituted β-D-glucopyranosiduronic acid, β-D-glucopyranose, α-L-rhamnopyranose, β-D-galactopyranose, β-D-xylopyranose, α-L-arabinofuranose, β-D-quinovopyranose, β-D-apiofuranose, gentibiose, chinovopyranose, fucopyranosyl, β-D-galactopyranosiduronic acid, β-D-ribopyranose, β-D-xylofuranose, β-D-allopyranose, β-D-arabinopyranose and β-D-apiopyranose.

7. Compounds according to one of claims 1 to 6, wherein **R^{N1}** is selected from hydrogen or from optionally substituted methoxy, acetyl and succinyl.

8. A compound of claim 1 which is selected from the group consisting of
(3S,18R,20S)-11-oxo-3-[(2,3,4,6-tetra-*O*-acetyl-beta-D-glucopyranosyl) methoxamino]-olean-12-en-29-oic acid, diphenylmethyl ester,
(3S,18R,20S)-11-oxo-3-[(2,3,4,6-tetra-*O*-acetyl-beta-D-glucopyranosyl) methoxamino]-olean-12-en-29-oic acid,
(3S,18R,20S)-11-oxo-3-[(beta-D-glucopyranosyl)methoxamino]-olean-12-en-29-oic acid,
(3S,18R,20S)-3-[2-S-(methyl-2,3,4-tri-*O*-acetyl-1-thio-beta-D-glucopyranosyluronate)-ethyl-amino]-11-oxo-olean-12-en-29-oic acid, diphenylmethyl ester,
(3S,18R,20S)-3-[2-S-(methyl-2,3,4-tri-*O*-acetyl-1-thio-beta-D-glucopyranosyluronate)-ethyl-amino]-11-oxo-olean-12-en-29-oic acid,
(3S,18R,20S)-3-[2-S-(methyl-1-thio-beta-D-glucopyranosyluronate)-ethyl-amino]-11-oxo-olean-12-en-29-oic acid,
(3S,18R,20S)-3-[2-(beta-D-glucopyranuronosylthio)-ethyl-amino]-11-oxo-olean-12-en-29-oic acid,
(3S,18R,20S)-3-{*N*-acetyl-N-[2-S-(methyl-2,3,4-tri-*O*-acetyl-1-thio-beta-D-glucopyranosyluronate)-ethyl]-amino}-11-oxo-olean-12-en-29-oic acid, diphenylmethyl ester,
(3S,18R,20S)-3-{*N*-acetyl-*N*-[2-S-(methyl-2,3,4-tri-*O*-acetyl-1-thio-beta-D-glucopyranosyluronate)-ethyl]-amino}-11-oxo-olean-12-en-29-oic acid,
(3S,18R,20S)-3-{*N*-acetyl-*N*-[2-S-(methyl-1-thio-beta-D-glucopyranosyluronate)-ethyl]-amino}-11-oxo-olean-12-en-29-oic acid,
(3S,18R,20S)-3-{*N*-acetyl-*N*-[2-(beta-D-glucopyranuronosylthio)-ethyl]-amino}-11-oxo-olean-12-en-29-oic acid,
(3S,18R,20S)-3-{*N*-(4-methoxy-1,4-dioxobutyl)-N-[2-S-(methyl-2,3,4-tri-*O*-acetyl-1-thio-beta-D-glucopyranosyluronate)-ethyl]-amino}-11-oxo-olean-12-en-29-oic acid, diphenylmethyl ester,
(3S,18R,20S)-3-{*N*-(4-methoxy-1,4-dioxobutyl)-N-[2-S-(methyl-2,3,4-tri-*O*-acetyl-1-thio-beta-D-glucopyranosyluronate)-ethyl]-amino}-11-oxo-olean-12-en-29-oic acid,
(3S,18R,20S)-3-{*N*-(4-methoxy-1,4-dioxobutyl)-N-[2-S-(methyl-1-thio-beta-D-glucopyranosyluronate)-ethyl]-amino}-11-oxo-olean-12-en-29-oic acid and
(3S,18R,20S)-3-{*N*-(4-methoxy-1,4-dioxobutyl)-N-[2-(beta-D-glucopyranurosylthio)-ethyl]-amino}-11-oxo-olean-12-en-29-oic acid

9. Compounds, or the pharmacologically effective salts thereof, according to one of the claims 1 to 8, as medicaments.

10. Pharmaceutical preparation, containing as active substance one or more compounds of general Formula I according to one of the claims 1 to 8, or the pharmacologically effective salts thereof, optionally in combination with conventional excipients and/or carriers.

11. Use of compounds of general Formula I according to one of the claims 1 to 8 for preparing a medicament for the treatment and/or prevention of chronic inflammatory diseases, autoimmune diseases, skin diseases, bone diseases, metabolic diseases, infectious diseases and cancer.

12. Use of compounds of general Formula I according to claim 11 for preparing a medicament for the treatment and/or prevention of infectious diseases.
